# EUROPEAN PATENT SPECIFICATION

(11) **EP 2 656 048 B1**
(45) Date of publication and mention of the grant of the patent: **25.11.2020**
(21) Application number: 11850377.0
(22) Date of filing: 16.12.2011
(51) Int. Cl.: G01N 21/05, G01N 35/08, G01N 21/27, G01N 33/53, G01N 21/45, G01N 21/77, G01N 21/55, G01N 35/00

(54) **DISPERSION INJECTION METHODS FOR BIOSENSING APPLICATIONS**
DISPERSIONSINJEKTIONSVERFAHREN FÜR BIOSENSORISCHE ANWENDUNGEN
PROCÉDÉS D'INJECTION PAR DISPERSION POUR DES APPLICATIONS DE BIODÉTECTION

(30) Priority: 23.12.2010 US 201061426665 P; 02.12.2011 US 201161566502 P
(43) Date of publication of application: 30.10.2013
(73) Proprietor: Molecular Devices, LLC, San Jose, CA 95134 (US)
(72) Inventor: QUINN, John, Gerard, Edmond OK 73034 (US)
(74) Representative: Hoeger, Stellrecht & Partner Patentanwälte mbB
(86) International application number: PCT/US2011/065613
(87) International publication number: WO 2012/087840

(56) References cited:
- WO-A1-2009/025680
- US-A1- 2003 095 897
- US-A1- 2005 089 890
- US-A1- 2007 084 940
- US-A1- 2007 261 479
- US-A1- 2010 196 205
- OSTERGAARD J & JENSEN H: "Simultaneous Evaluation of Ligand Binding Properties and Protein Size by Electrophoresis and Taylor Dispersion in Capillaries", ANALYTICAL CHEMISTRY, AMERICAN CHEMICAL SOCIETY, US, vol. 81, no. 20, 15 October 2009 (2009-10-15), pages 8644-8648, XP002637139, ISSN: 0003-2700, DOI: 10.1021/AC901419X [retrieved on 2009-09-23]
- UPMA SHARMA ET AL: "Diffusivity of Solutes Measured in Glass Capillaries Using Taylor's Analysis of Dispersion and a Commercial CE Instrument", ANALYTICAL CHEMISTRY, vol. 77, no. 3, 1 February 2005 (2005-02-01), pages 806-813, XP55113297, ISSN: 0003-2700, DOI: 10.1021/ac048846z
- RICH R L ET AL: "Biosensor-based fragment screening using FastStep injections", ANALYTICAL BIOCHEMISTRY, ACADEMIC PRESS INC, NEW YORK, vol. 407, no. 2, 15 December 2010 (2010-12-15), pages 270-277, XP027354975, ISSN: 0003-2697 [retrieved on 2010-08-25]
- ANNA BIELEJEWSKA ET AL: "Evaluation of Ligand-Selector Interaction from Effective Diffusion Coefficient", ANALYTICAL CHEMISTRY, vol. 82, no. 13, 1 July 2010 (2010-07-01), pages 5463-5469, XP055113300, ISSN: 0003-2700, DOI: 10.1021/ac1008207
- SHANK-RETZLAFF MARY L ET AL: "Analyte gradien-surface plasmon resonance: a one-step method for determining kinetics tares and macromolecular binding affinities", ANALYTICAL CHEMISTRY, AMERICAN CHEMICAL SOCIETY, US, vol. 72, no. 17, 1 September 2000 (2000-09-01), pages 4212-4220, XP002994633, ISSN: 0003-2700, DOI: 10.1021/AC0001030

## Description

Biosensors are commonly used to perform kinetic studies of complex molecular interactions such as those between drug-target, hormone-receptor, enzyme-substrate and antigen-antibody. The biosensors are typically in a flow injection-based fluidic system wherein one or more sensing regions are housed within a flow cell conduit of the fluidic system. The fluidic system further defines one or more flow channel conduits that direct fluid flow to the sensing regions in the flow cell conduit. The sensing region provides surfaces that support immobilized molecules referred to generally as "ligands." The ligands are potential binding partners for molecules known as "analytes" which are present in fluids that are directed to the sensing region of the flow cell conduit via the flow channel conduit. Typically one member of an affinity pair, the ligand, is immobilized onto a surface in the sensing region while the second member, the analyte, is exposed to this ligand-coated surface for sufficient time to form analyte-ligand complexes at the sensing region. The accumulation of the resulting affinity complexes at the sensing region is detected by a label-free detection method selected from the group consisting of evanescent filed-based optical refractometers, surface plasmon resonance, optical interferometers, waveguides, diffraction gratings, photonic crystal waveguide arrays, and gravimetric microbalances based on frequency dampening of piezoelectric substrates. The biosensor response is then plotted in real-time and is referred to hereafter as a response curve or binding response curve. In order to accurately determine the affinity complex interaction parameters (binding affinity constants, association constants, dissociation constants, diffusion coefficients, etc), the ligand should be exposed to multiple analyte concentrations.

The standard analyte sample injection method, known as fixed concentration injections (FCI's), involves separate preparation and injection of a series of discrete analyte samples each containing different concentrations of a given analyte. The analyte concentrations are chosen to provide a range of analyte concentration of approximately two to three orders of magnitude (eg., 1 nM, 3 nM, 9 nM, 18 nM, 54 nM, 162 nM etc.). Since each analyte concentration is separately prepared and injected as a discrete sample, the assay is complex, time consuming, and provides many opportunities for human and systematic error. Moreover, many FCI protocols call for removal of the bound analyte after each analyte sample is injected. Thus, the FCI method (1) is time consuming resulting in low throughput; (2) provides greater opportunity for variation in analysis temperature, ligand degradation, analyte degradation and evaporative loss; (3) provides greater opportunity for human error; and (4) increases the potential for systematic errors associated with volume handling due to the high number of fluid manipulations required. These problems ultimately accumulate in the recorded data and interfere with data analysis causing higher error in the determination of interaction parameters.

The primary benefit of using the FCI method is that the analyte concentration of each separate injection is presumably known (assuming no error in the preparation of the dilution series). This is important since an inaccurate analyte concentration translates directly into an equivalent error in the estimation of affinity interaction constants. However, accurate representation of analyte concentration has been the primary deficit in alternative methods developed to solve some of the problems associated with FCI. These alternative injection methods utilize a single analyte sample injection that is manipulated to generate a range of analyte concentrations prior to or within a fluidic system en route to the sensing region. The analyte concentrations can be roughly estimated in these single injection methods by applying a concentration calibration method performed under conditions that closely match the assay conditions. However, this calibration can only be applied to an assay performed under matching conditions (e.g. flow rate, temperature, ionic strength, viscosity, analyte molecular weight). In practice, this is a highly restrictive requirement that hinders the adoption of these alternative injection methods. Thus, the prior art has yet to provide a general method to accurately determine the entire range of analyte concentrations produced under variable assay conditions thereby avoiding the need for special case calibration methods. The methods described herein solve this problem and others by providing a single injection method that accurately models the actual analyte concentration within the flow cell over the entire range of analyte concentrations as the injection progresses over the sensing region.

Rich R L et al.: "Biosensor-based fragment screening using FastStep injections", Analytical Biochemistry, 407 (2010) 270-277 discloses an analyte injection method for the SensiQ Pioneer surface plasma resonance-based biosensor referred to as "FastStep". It discloses a FastStep threefold (five steps) and a two fold (seven steps) dilution injection series of 20% sucrose. The profile of the sucrose injection is used to define the concentration of an analyte throughout the entire concentration profile

In-M:LShank-Ftetzlaff et Al., Anal. Chem. 2000, Vol.72, pp.4212-4220, a gradient marker is used to produce a linear gradient of analyte concentration over a sensor surface and the rate at which the analyte binds to immobilized receptors is measured and fitted with a model so as to determine the kinetic rate and the binding affinity associated with the analyte and the receptor.

The invention is defined in the claims.

An injection method is provided that comprises the use of a single injection to produce a well-defined analyte concentration gradient by optimizing the physical dispersion process. The method further comprises the use of a dispersion term that is integrated into the desired binding interaction model for analysis of the analyte-ligand interaction, wherein the dispersion term reflects the physical dispersion process thereby accurately defining the analyte concentrations at the sensing region for any time during the injection.

In one embodiment, a sample injection method for determining the affinity interaction parameters of analyte-ligand interactions in a biosensor is provided. The biosensor in which the method is performed preferably comprises a flow channel conduit in fluid communication with a flow cell conduit, the flow cell conduit housing at least one sensing region having at least one ligand immobilized thereon. The method comprises injecting a fluid sample containing an analyte through the flow channel conduit to the flow cell conduit under conditions sufficient to cause dispersion of the analyte. The dispersion results in the formation of an analyte concentration gradient having a maximum analyte concentration and a minimum analyte concentration that differ by at least one order of magnitude. Additionally, the analyte concentration in the gradient is continuously changing between the maximum analyte concentration and minimum analyte concentration. The measured biosensor responses elicited by the analyte interacting with the ligand at the sensing region are measured as the analyte concentration gradient progresses through the flow cell conduit. The responses measured at the sensing region provide a binding response curve. A dispersion term that accounts for the dispersion conditions present during the injection is selected to represent the analyte concentration present in the flow cell conduit at any time point during the injection. The dispersion term is incorporated into a binding interaction model which is fitted to the binding response curve thereby allowing determination of the affinity interaction parameters including the affinity constant and the kinetic interaction constant.

The invention will be further described, by way of example, with reference to the accompanying drawings, in which:
FIG. 1 is a schematic illustration of a flow channel conduit and flow cell conduit configuration in a biosensing system.
FIG. 2A depicts a representative cross-sectional analyte concentration profile along the flow channel conduit arising from a pulse Taylor dispersion injection.
FIG. 2B is a schematic illustration of the inhomogenous analyte distribution that evolves as the injected analyte progresses through a flow channel conduit defining the pulse Taylor dispersion condition.
FIG. 2C depicts overlaid analyte binding response curves for pulse Taylor dispersion injection of nine serial ten-fold dilutions of analyte while the expected analyte concentration during these injections is indicated by the dotted peak.
FIG. 3A depicts the average cross-sectional analyte concentration profile along the flow channel conduit arising from a sigmoidal Taylor dispersion injection.
FIG. 3B is a schematic illustration of the inhomogenous analyte distribution that evolves as the injected analyte progresses through a flow channel conduit defining the sigmoidal Taylor dispersion condition.
FIG. 3C depicts overlaid analyte binding response curves for sigmoidal Taylor dispersion injection of nine serial ten-fold dilutions of analyte while the expected analyte concentration during these injections is indicated by the dotted peak.
FIG. 4A depicts overlaid response curves representing the negligible response elicited over a non-coated sensing surface (no ligand) by pulse Taylor dispersion injections of a series of two-fold serial dilutions of warfarin (0.244 mM to 1 mM).
FIG. 4B depicts overlaid response curves representing warfarin (analyte)-human serum albumin (ligand) binding elicited by triplicate pulse Taylor dispersion injections of a series of two-fold serial dilutions of warfarin (0.244 mM to 1 mM).
FIG. 4C depicts three overlaid binding response curves representing the warfarin (analyte)-human serum albumin (ligand) binding response curve (binding isotherm) elicited by a pulse Taylor dispersion injection of warfarin (1 mM neat concentration) plotted with the expected relative warfarin concentration profile (i.e. Gaussian-like peak) calculated from the appropriate dispersion term.
FIG. 4D is a standard two site steady-state affinity isotherm. The plot was created by plotting a set of response points selected from a single warfarin binding curve from FIG. 4C with respect to the expected warfarin concentration profile.
FIG. 4E are overlaid binding response curves representing warfarin (analyte) binding to human serum albumin (ligand) as analyzed by sigmoidal Taylor dispersion injections at a neat concentration of 1 mM. The expected warfarin concentration profile was calculated from the appropriate dispersion term and appears shifted to the right of the warfarin binding curves. A three-site real-time isotherm model was fitted directly to the real-time binding response curve (smooth curve overlying the binding response curve).
FIG. 5 depicts overlaid binding response curves representing a time progression series of curves for warfarin (analyte) binding to human serum albumin (ligand) using sigmoidal Taylor dispersion injections of 3 mM warfarin (neat concentration) repeated fifteen times at 15 minute intervals. The inset represents a derivative of the first seven replicate binding curves after a five point smoothing and shows three distinct binding site populations (I, II, II).
FIG. 6A depicts overlaid response curves representing the bulk refractive index response for pulse Taylor dispersion injections of vancomycin (10 mg/ml) repeated in quadruplicate and fitted to the dispersion term (Equation 1) (smooth curves overlying the recorded bulk refractive index curve).
FIG. 6B is a plot of the analyte diffusion coefficients calculated from the bulk refractive index response (*D_{M}*) for twenty different biomolecules (under similar conditions described with respect to FIG. 6A) with respect to the expected diffusion coefficients (*D_{M}*) with a regression line fitted.
FIG. 7A depicts overlaid binding response curves for binding of ScFv (analyte) to rhErbB2/Fc (ligand) for standard fixed concentration injections using 10-fold dilutions of ScFv (1000 nM to 0.1 mM). A simple kinetic interaction model was fitted (smooth curves) with global constraint of kinetic parameters and superimposes upon the experimental curves.
FIG. 7B depicts overlaid binding response curves for binding of ScFv (analyte) to rhErbB2/Fc (ligand) for sigmoidal Taylor dispersion injections using 10-fold dilutions of ScFv (1000 nM to 0.1 mM). A simple kinetic interaction model that includes a dispersion term has been fitted (smooth curves) with global constraint of kinetic parameters and superimposes upon the experimental curves.
FIG. 8A depicts overlaid binding response curves for binding of furosemide (analyte) to carbonic anhydrase II (ligand) for fixed concentration injections of three-fold serial dilutions of furosemide (0.46 µM to 111 µM) with a fitted 1:1 kinetic interaction model and global constraint of kinetic parameters.
FIG. 8B depicts overlaid binding response curves for binding of furosemide(analyte) to carbonic anhydrase II (ligand) by sigmoidal Taylor dispersion injections repeated at varying starting concentrations of furosemide (ten-fold serial dilutions from 1 µM to 1 mM) with a fitted 1:1 kinetic interaction model that includes a dispersion term and global constraint of kinetic parameters.
FIG. 8C is a binding response curve for furosemide(analyte) binding to partially denatured carbonic anhydrase II (ligand) by sigmoidal Taylor dispersion injections repeated at varying starting concentrations of furosemide (three-fold serial dilutions from 12 µM to 1 mM) and globally fitted to a 2:1 kinetic interaction model that included a dispersion term The injections were performed at 10°C to preserve weak non-specific binding to the partially denatured ligand in order to mimic a realistic complex data set.

The methods described herein are related to a dispersion-based injection method that solves the technical limitations of prior art, namely the ability to accurately represent the analyte concentrations in a dispersion-generated analyte concentration gradient for label-free biomolecular interaction analysis. As used herein, the term "dispersion" means a mixing process that causes the analyte concentration to become inhomogenous with respect to the cross-section and length of the flow channel conduit into which the analyte-containing sample is injected. Dispersion is due to the combined action of analyte diffusion towards the walls of the capillary (flow channel conduit) and the parabolic velocity profile within the capillary that results from convective flow through the capillary. Thus, the profile of an analyte concentration gradient created by dispersion is a complex function of the physical conditions present during the injection, such as temperature, applied forces (e.g. gravity), molecular weight, molecular shape, liquid viscosity, ionic strength, isoelectric point, flow channel geometry and flow rate. In biomolecular interaction analysis, an accurate representation of the analyte concentration is important since the binding interaction at the sensing region is influenced by the availability of analyte (i.e. analyte concentration). Accordingly, the actual analyte concentration gradient profile that passes over the sensing region contains information that defines the preceding analyte dispersion process and therefore becomes encoded or "imprinted" into the dependent analyte-ligand binding response curve.

Therefore, the methods described herein incorporate a relevant dispersion expression (dispersion term) into the binding interaction model of interest to accurately define the analyte concentration at the sensing region as a function of time. Additionally, the method involves the use of injection conditions that are sufficient to create a dispersion profile that is accurately represented by the dispersion term. An injection method that is performed under the physical conditions to comply with a particular dispersion theory not only provides an extended analyte concentration range, but also accounts for many physical parameters that affect dispersion such as the analyte diffusion coefficient. The inclusion of the analyte diffusion coefficient as a fitted parameter in the inventive method significantly extends biophysical characterization by label-free biosensors beyond what has been possible to date. Taken together, the methods described herein involve performing an analyte sample injection under physical conditions sufficient to cause the analyte to undergo a dispersion event that is in compliance with the assumptions and conditions under which the selected dispersion term is derived. This method can be implemented without compromising other assay performance parameters such as throughput, robustness, reproducibility and resolution, although parameters can be modified if necessary for a particular assay.

In one embodiment, the method is performed in a biosensor comprising a flow channel conduit that is in fluid connection with a flow cell conduit downstream from the analyte injection point. The flow channel conduit is preferably filled with a carrier fluid free of analyte. The flow cell conduit houses at least one sensing region having one or more ligands immobilized thereon. A sample comprising a volume of carrier fluid containing the analyte is injected into the flow channel conduit such that the analyte undergoes dispersion upon continued flow through the flow channel conduit en route to the flow cell conduit. The injection conditions, including the flow channel conduit geometries, are optimized to produce a dispersion profile that is accurately represented by a dispersion term. The dispersion event produces an analyte concentration gradient that passes through the flow cell conduit where the formation of affinity complexes (analyte-ligand interactions) is measured thereby generating a binding response curve. The dispersion term is included in a binding interaction model that is fitted to the binding response curve where the dispersion term represents the analyte concentrations at the sensing region as a function of injection time. The inclusion of this dispersion term obviates the need for special case calibration by providing a realistic physical description of the dispersion process and hence, provides reliable representation of analyte concentration in the binding interaction model.

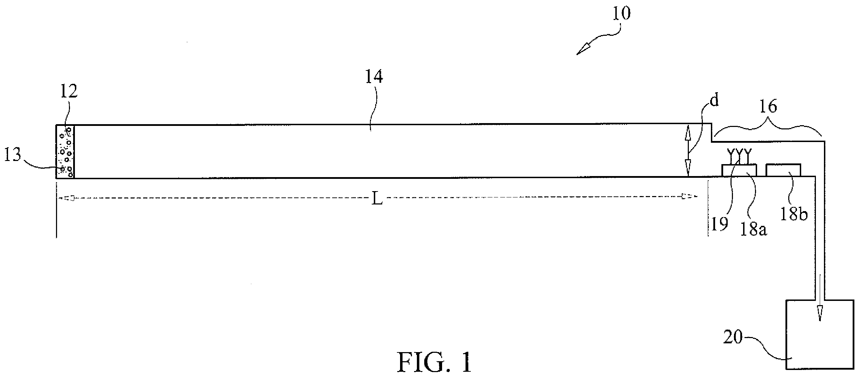

Referring now to FIG. 1, an example of a fluidic configuration 10 of a biosensor for use in connection with various aspects of the current invention is depicted. An analyte 13 contained in an initial sample volume 12 is made to enter a flow channel conduit 14 by pressure driven flow. The flow channel conduit 14 is of a length L and diameter d sufficient to produce a desired analyte dispersion profile. The flow channel conduit 14 leads to a flow cell conduit 16 that houses one or more sensing regions 18 interrogated by a label-free transducer such as a surface plasmon resonance detector (not shown). One or more sensing regions 18a have ligands 19 immobilized thereon. The response elicited by the interaction between the analyte 13 and ligand 19 is measured at the sensing region to produce a binding response curve. Additionally, one or more sensing regions 18b can be free of immobilized ligand 19 and can be used to measure the bulk refractive index of the fluid sample 12. The sample 12 proceeds to waste 20 after passing through the flow cell conduit 16.

In one embodiment, the volume of the flow cell conduit is small relative (<1%) to the flow channel conduit allowing dispersion within the flow cell conduit to be neglected when calculating the analyte concentration within the flow cell. If the volume of the flow cell conduit is significant (>1%) relative to the flow channel conduit, then the volume and geometry of the flow cell must be considered as an extension of the flow channel conduit when calculating the analyte concentration. In this case, the effective flow channel dispersion geometry includes terms that account for both the flow channel conduit geometry and the flow cell conduit geometry. Furthermore, the flow cell conduit may be part of the flow channel conduit where a defined boundary between these channels is absent. For example, the sensing regions may be disposed within the flow channel conduit itself where the flow channel geometry is consistent along its length. In practice the flow cell conduit is typically defined by a lower channel height in order to support high mass transport of analyte across the non-stirred boundary layer and onto the sensing surface. However, it is entirely possible to construct a flow channel conduit with a channel height that supports high mass transport rates. Lowering the height of the flow channel conduit would necessitate higher operating pressures, but would be expected to reduce the dispersion time. This may be appropriate where fast analysis of higher molecular weight analytes is required.

The embodiments described herein are described using a fluidic system where the various elements are connected in free space. For example, the flow channel conduit is a tube or capillary that is brought into fluid connection with a flow cell conduit using conventional free space adapters/fittings. However, it should be understood that the method can be performed using alternative designs wherein the flow channel conduit and flow cell conduit are integrated into a single miniature planar microfluidic device which offers practical advantages such as fabrication reproducibility. The dispersion terms can be modified to account for the approximately rectangular flow channel cross sections typical of planar microfluidic devices.

In one aspect, the flow channel conduit is composed of a capillary of uniform circular cross-section and is drawn from an inert material such as PEEK, Teflon, Teflon derivatives, or glass, although other materials could be acceptable. The use of soft elastic materials which allows undesirable variation in geometry with respect to pressure (e.g. pump pulsation) should be avoided. However, in certain special case applications, the use of such compliant materials might be exploited to modulate the analyte dispersion profile. A more precise modulation of dispersion characteristics may be produced by making precise adjustments to the geometry of the flow channel conduit. Adjustment of the geometry may be performed on-the-fly as the dispersion is in progress or may be performed in advance of the dispersion injection. The volume of the dispersion tube dictates the amount of sample consumed and the TDi method can be practiced with dispersion tubes (flow channel conduits) over a wide volume range. However, to reduce sample consumption and with consideration of operational backpressure, suitable limits for the volume of the dispersion tube are from about 10 µL to about 2000 µL and assuming the dispersion tube diameter ranges from about 0.05 mm to 1.0 mm.

The current method can also be practiced with the incorporation of additional actuators, heaters or sensors. This may be considered in order to further modulate the analyte dispersion characteristics. The volume and effective diameter of the flow channel conduit, injection flow rate and analyte molecular weight are interrelated parameters with many possible combinations that meet the requirements of the Taylor dispersion theory. Parameter combinations that are not consistent with dispersion theory are avoided by employing upper and lower flow rate limits for the combination of dispersion parameters presented by the experiment. However, specific limits can be defined by dimensionless parameters. It is desirable to choose a flow channel conduit volume/diameter and operating pressure that minimizes sample consumption for a given analyte class (e.g. proteins, drugs, particles) while providing adequate throughput. An optimal compromise between these parameters is not difficult to achieve for low molecular weight analytes (e.g. drug sized compounds), but higher molecular weight analytes such as proteins require an injection time of 10-15 minutes in low pressure fluidics system.

For example, the flow channel conduit can be actively heated or cooled to modify the analyte diffusion coefficient thereby providing an additional means of controlling the analyte dispersion profile. The use of varying temperatures can provide thermodynamic analysis of interactions, improve resolution of the diffusion coefficient or other parameters, or permits accelerated dissociation of strong binders for increased throughput. Thermal denaturation of the reagents can limit these temperature based methods. Typically protein-based analytes can tolerate temperatures that do not exceed 60°C. However even among protein-based analytes there are exceptions to this limit. Drug like molecules, nucleic acids, and carbohydrate-based analytes are far more stable allowing temperatures to approach the boiling point of the carrier buffer (typically approximately 100°C at atmospheric pressure). Higher operating pressures enable even higher temperatures to be applied. Cryogenic agents such as glycerol can be added to the carrier fluid (i.e. sample buffer) to prevent freezing at lower temperatures. Therefore the absolute temperature range can be stated as any temperature where the carrier stream does not boil or freeze at the operating pressure within the flow channel conduit.

In one aspect, the flow cell conduit is typically a rectangular compartment with a high numerical aperture where the volume and height of the flow cell channel is minimized. The flow cell employed in the Example provided below were rectangular with approximate dimensions of length = 3 mm, width = 0.5 mm and height = 30 µm. However, other more complex geometries can be chosen in the practice of the dispersion method.

The pump used in connection with the current method should provide pulse-free pressure driven flow where variation in flow rate and pressure are minimal as this facilitates accurate modeling of the dispersion process. Syringe pumps are suitable, although peristaltic pumps may be adequate where absolute accuracy is not required.

In one aspect, the sensing regions are interrogated optically by a surface-sensitive refractometer based on surface plasmon resonance (SPR). Similar performance can be expected when using these surface sensitive evanescent-field based refractometers. Other optical detection principles such as interferometry are less sensitive to bulk refractive index and more specific to the mass loading event that manifests as a growing biofilm layer. These detectors are advantageous where interference from bulk refractive is undesirable. In practice SPR's sensitivity to both bulk refractive index and surface refractive index is advantageous as it is possible to verify dispersion parameters from the bulk refractive index response independently of the specific binding response. For example, analyte present at high concentrations often provides a bulk refractive index response that is adequate to determine the analyte diffusion coefficient and general dispersion parameters thereby allowing these parameters to be fitted as constants when applying the affinity model to the specific binding response curve. Alternative placement of the sensing regions such as at intervals along the flow channel conduit enable replicates of the same affinity interaction to be performed under different dispersion conditions providing a more comprehensive analysis of dispersion-dependent binding. When an analyte stream enters the flow cell conduit, binding to ligand immobilized at the sensing region will occur. This mass loading increases the average refractive index close to the sensing surface that is probed by the evanescent field. The evanescent field is created from the reflection of a wedge-shaped monochromatic beam (where the light source is an 880 nm light emitting diode) from the surface under conditions of total internal reflection. This results in a reflection minimum with respect to incidence angle when a thin noble metal film is present at the interface. In this instance, the metal is preferably gold due to its stability and ease by which it can be made to anchor biocompatible films or hydrogels that are suitable for affinity binding studies. The angular position of the reflectance minimum changes with changes in surface refractive index and are tracked using a photodiode array and a minimum hunt algorithm that provides a real-time location of the minimum. The detector is pre-calibrated to convert this real time location to an equivalent refractive index change which is then plotted as a function of time to create a real-time binding curve.

In one embodiment, the current method involves the injection of a fluid sample containing an analyte under conditions sufficient to cause dispersion in compliance with Taylor dispersion theory (hereinafter "Taylor dispersion injections" or "TDi"). As will be discussed in more detail below, there are two basic types of TDi's, a pulse TDi and a sigmoidal TDi. Various other types of dispersion injections can be performed so long as the dispersion term accounts for the conditions present during the sample injection.

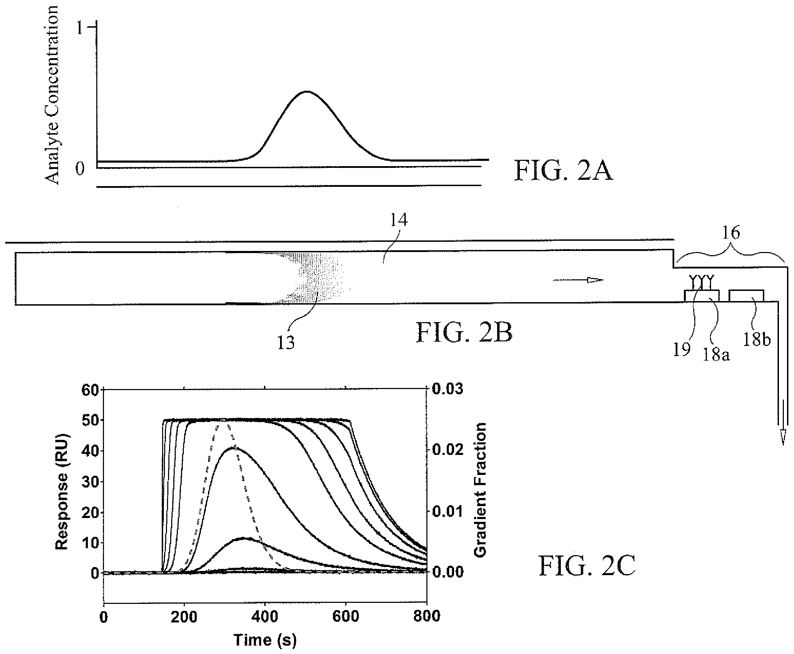

In one aspect, the method involves a TDi that results in a Gaussian-like gradient profile as depicted in FIG. 2A. This is referred to herein as a pulse TDi. Referring now to FIG. 2B, a relatively small sample volume, in one aspect, < 5% of the flow channel conduit capacity volume, is injected into the flow channel conduit 14 and is followed by analyte-free carrier liquid. In this embodiment, the analyte 13 is free to disperse with analyte-free carrier liquid on both tailing and leading fronts thereby forming the Gaussian-like peak, or pulse. In this aspect, the residence time (τ) defines the peak position with respect to injection time.

FIG. 2C shows nine simulated binding response curves for the binding of an analyte 13 over nine orders in neat analyte concentration to the ligand 19 at the sensing surface 18a using pulse TDi. Pulse TDi injections with high neat analyte concentrations give rise to binding curves that begin sooner than TDi injections performed using lower neat analyte concentrations. TDi shows that irrespective of concentration the binding curves remain resolved over nine orders and saturate at different times with the exception of low concentrations which give rise to binding that is inadequate to approach saturation at typical contact times (i.e. 1-10 min). This high resolving power over a very wide concentration range is not possible with standard FCI formats. FIG. 2C also shows the expected analyte concentration profile as calculated from Equations 1 and 2 (provided below) and indicates that the maximum analyte concentration represents a small fraction of the neat analyte concentration when using pulse TDi. The location of the peak maximum specifies the residence time (τ).

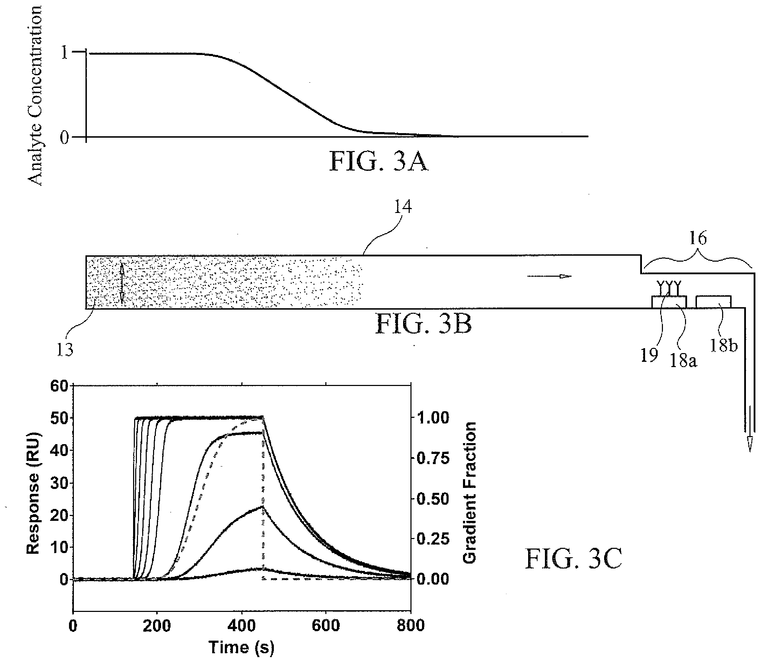

In an alternative aspect, the method involves a TDi that results in a sigmoidal gradient profile as depicted in FIG. 3A. This is referred to herein as a sigmoidal TDi. Referring now to FIG. 3B, the analyte sample is of a volume sufficient to displace all the analyte-free liquid that was contained in the flow channel conduit 14 before the injection commenced. The mixed liquid exiting the flow channel conduit containing the dispersed analyte sample will flow through the flow cell conduit as an increasing analyte concentration gradient that reaches a maximum concentration equal to the concentration of the initial sample analyte concentration. As the analyte 13 progresses through the flow channel conduit 14, the analyte concentration profile will take the form of an evolving sigmoidal gradient profile as depicted in FIG. 3A.

FIG. 3C shows nine simulated binding response curves for the binding of an analyte 13 over nine orders in neat analyte concentration to the ligand 19 at the sensing surface 18a using sigmoidal TDi. As was the case for Pulse TDi, this sigmoidal TDi shows that high neat analyte concentrations give rise to binding curves that begin sooner than TDi injections performed using lower neat analyte concentrations. Irrespective of concentration, the binding curves remain resolved over nine orders and saturate at different times with the exception of low concentrations which give rise to binding that is inadequate to approach saturation at typical contact times (i.e. 1-10 min). FIG. 3C also shows the expected sigmoidal analyte concentration profile as calculated from Equations 2, 3 and 4 (provided below) and indicates that the maximum analyte concentration within the flow cell conduit approaches the neat analyte concentration. The location of the inflection point (i.e. time at half maximum concentration) of the sigmoidal specifies the residence time (τ). This residence time is equal to the time required to inject a sample volume equal to the total volume of the flow channel conduit. For example, a flow channel conduit with a capacity volume of 50 µL will yield a residence time of one minute when the sample is injected at 50 µL/min. This assumes that the injected analyte sample does not interact with the walls of the flow channel conduit 14 and is not retarded relative to the convective flow of the carrier liquid.

In either aspect of the current embodiment, the injection conditions sufficient to cause Taylor dispersion of the analyte are determined in accordance with the following rules:
1. Laminar flow must predominate inside the flow channel conduit.
2. For a pulse TDi (discussed below), the fluid sample containing analyte possesses a volume that is less than 10%, and preferably less than 5%, of the total capacity volume of the flow channel conduit. For sigmoidal TDi, the injected sample volume should be in the range from half the volume of the flow channel conduit volume to a maximum equivalent of the total flow channel volume. A sample volume below the low limit (half the volume) will produce partial sigmoidal TDi gradients that do not approach the maximum expected concentration (i.e. concentration of the neat sample). While such gradients may be useful, it is optimal to approach a stable concentration before the sigmoidal TDi injection is terminated since the resulting response curves defines the dispersion process more thoroughly.
3. The flow channel conduit is positioned horizontally to avoid the influence of gravity on the liquid flow with the flow channel conduit.
4. If the flow channel conduit must be coiled, then the coiling radius is greater than 1000^{∗}*d*, where *d* is flow channel conduit diameter.
5. The lower flow rate limit or average velocity of the injected sample is defined by the Peclet number (N*_{Pe}*) and must be greater than 500:
where ${N}_{Pe} = \left(d*u\right) / D$ where

| | |
|---|---|
| *d* | = flow channel conduit diameter (m); |
| *u* | = average velocity of fluid (m s⁻¹); and |
| *D* | = analyte diffusion coefficient (m² s⁻¹) |

6. The upper flow rate limit as defined in terms of dimensionless time (*τ*') is greater than 3.0:
where $τ ′ = D * τ / {\left(0.5*d\right)}^{2}$ where

| | |
|---|---|
| *τ* | = mean analyte residence time (s) or *Uu;* |
| *L* | = length of capillary (m); |
| *u* | = average velocity of fluid (m s⁻¹); |
| *d* | = flow channel conduit diameter (m); and |
| *D* | = analyte diffusion coefficient (m² s⁻¹). |

In order to determine if a given set of injection conditions will conform to Taylor dispersion theory for a particular analyte, the molecular weight of the analyte can be used to provide an estimate of the analyte diffusion coefficient thereby enabling calculation of the N*_{Pe}* and *τ*'. While values of *τ*' should exceed 3 values that are in excess of 10 simply prolong the injection time with little added benefit while unnecessarily decreasing throughput. It is possible to establish proper dispersion conditions at the outset thereby eliminating the need for preliminary calibration steps to ensure the conditions are consistent with Taylor dispersion theory. The Stokes-Einstein-Sutherland equation can be used to calculate the diffusion coefficient D based on the molecular weight *M_{w}* (also referred to herein as *Mᵣ*) of the analyte. The Stokes-Einstein-Sutherland equation is as follows: $D = {k}_{b} T / 6 {πμR}_{0} \left(f/{f}_{0}\right)$ where k_{b} is Boltzmann's constant (1.381x 10⁻²³ J/K), T is temperature (Kelvin), µ is buffer viscosity (-0.001kg/s.m), and Ro is the analyte radius (m)and *f*/*f*₀ is the analyte friction factor (usually 1.2). The analyte radius can be estimated from the M_{w} as follows: ${R}_{0} = \sqrt[3]{\frac{3 MwV}{4 π 6.023 e 23}}$ Alternatively, the diffusion coefficient can be experimentally determined by measuring the bulk refractive index of the desired analyte as described in Example 6 provided below.

In the pulse TDi embodiment, the dispersion term selected is preferably represented by the following equation: $C \left(t\right) = \frac{2 {C}_{in} {V}_{i}}{{π}^{3 / 2} {d}^{2} \sqrt{kt}} exp \left[-\frac{0.25 {L}^{2} {\left(1-t/τ\right)}^{2}}{kt}\right]$ where

| | |
|---|---|
| *C(t)* = | analyte concentration at detector (mol m⁻³) |
| *Cᵢₙ* = | concentration of analyte injected (mol m⁻³) |
| *Vᵢ* = | sample injection volume (m³) |
| *d* = | capillary (flow channel conduit) diameter (m) |
| *τ* = | mean analyte residence time (s) or *L*/*u* |
| *L* = | length of capillary (m) |
| *u* = | average velocity of fluid (m s⁻¹) |
| *k* = | Taylor-Aris dispersion coefficient (m² s⁻¹) which is represented by the following equation: |
| $α \frac{{u}^{2} {d}^{2}}{192 D} + D$ | |

where

| | |
|---|---|
| *D* = | analyte diffusion coefficient (m² s⁻¹) and |
| α = | conduit shape factor |

The value 1/192 is a scaling factor defined by the circular cross-section of the flow channel conduit and can be calculated to account for other cross-sectional geometries. A conduit shape factor, α, is used to compensate for any departures from the idealized cross-sectional flow channel conduit geometry. TDi analysis of a standard analyte, such as sucrose, can be used to determine α by constraining the other parameters and solving for α by fitting equations 1 and 2. By including a standard sucrose TDi curve, it is possible to estimate α while also verifying *Vᵢ* by integration of the area under the pulse curve. This standard analyte is typically present at a concentration sufficient to produce a measureable bulk refractive index response and surface binding is not required. Direct fitting of the TDi equations to this bulk refractive index response provides verification of dispersion parameters in the absence of any affinity binding response. In some applications (e.g. fragment screening), the specific analyte is present at sufficiently high concentrations to produce an analyte bulk refractive index dispersion in addition to a specific affinity binding response to the ligand-coated surface. The bulk refractive index response curve is isolated from the response curve recorded over a non-coated surface allowing the analyte diffusion coefficient and concentration to be determined before fitting the affinity binding model to the referenced affinity binding response curve. It should be noted that the concentration is easily calculated from the analyte bulk refractive index since the refractive index increment of a compound is readily calculated from the known structure.

In practice, most flow channel conduits tested returned α ≅ 1.0 indicating that the assumption of idealized circular flow channel geometry was valid. Interestingly, it is possible to optimize the cross-sectional geometry of the flow channel conduit to decrease the impact of diffusion on the dispersion gradient profile. This allows for producing matching dispersion profiles for analytes of different molecular weight.

In the sigmoidal TDi embodiment, the dispersion term selected is represented by Equation 3 below for the injection time t that is less than *τ* and by Equation 4 below for the injection time t that is greater than *τ* : $\begin{matrix}C \left(t\right) = \frac{{C}_{in}}{2} \left[1-erf\frac{1 - \frac{t}{τ}}{2 \sqrt{\frac{k}{uL} \frac{t}{τ}}}\right] & \left(t<τ\right)\end{matrix}$ $\begin{matrix}C \left(t\right) = \frac{{C}_{in}}{2} \left[1+erf\frac{1 - \frac{t}{τ}}{2 \sqrt{\frac{k}{uL} \frac{t}{τ}}}\right] & \left(t>τ\right)\end{matrix}$ where

| | |
|---|---|
| *C(t)* | = analyte concentration at detector (mol m⁻³) |
| *Cᵢₙ* | = concentration of analyte injected (mol m⁻³) |
| *τ* | = mean analyte residence time (s) or *L*/*u* |
| *L* | = length of capillary (m) |
| *u* | = average velocity of fluid (m s⁻¹) |
| *k* | = Taylor-Aris dispersion coefficient (m² s⁻¹) (*Equation 2*) |
| erf | = Gauss error function |

The sigmoidal TDi and pulse TDi embodiments are each capable of generating smoothly varying analyte concentration gradients over 3-4 orders in magnitude which is suitable for label-free biosensing. However, the maximum concentration of analyte in the pulse TDi approach is significantly less than the analyte concentration in the initial sample (neat analyte concentration) while the maximum concentration of analyte in the sigmoidal TDi approach is equal to the neat analyte concentration.

Furthermore, Equations 1-4 can be supplemented by terms that describe diffusion in the axial direction, thereby extending the conditions under which dispersion can be accurately modeled. Although the analysis presented is confined to rising analyte concentration gradients, it is evident that the TDi method can be reversed to create decaying gradients or even combined en route to the flow cell to create more complex gradients. In the case of sigmoidal TDi, the sample entering the flow channel conduit may have undergone a previous dispersion event in another flow channel conduit and the accumulative effect of dispersion within two different flow channel conduits connected in series can be exploited to change the dispersion profile. Furthermore the previous concentration gradient might be created with minimal dispersion effects by combining sample and carrier buffer streams before entering the flow channel conduit.

Various binding interaction models can be used in conjunction with the dispersion methods described herein. In one aspect, the two-compartment 1:1 pseudo-first-order kinetic interaction model is utilized. This model is composed of two differential equations that describe the change in the concentration of affinity complexes *(dR*/*dt)* at a sensing region and the analyte concentration gradient (*dC*/*dt*) as the analyte passes from the bulk carrier liquid through the diffusion boundary layer within the flow cell conduit onto the sensing region. $\frac{dC}{dt} = \left(-{k}_{a}C\left({R}_{max}-R\right)+{k}_{d}R+{k}_{m}\left({C}_{in}-C\right)\right)$ $\frac{dR}{dt} = {k}_{a} C \left({R}_{max}-R\right) - {k}_{d} R$ where

| | |
|---|---|
| *R* | = biosensor response (response units (RU)), |
| *Rₘₐₓ* | = maximum response expected if all ligand sites are occupied (RU) |
| *Cᵢₙ* | ± injected analyte concentration (M), which becomes zero when the dissociation phase begins. |
| *C* | = concentration of the analyte at the sensing surface (M) |
| *kₘ* | = mass transport constant (RU M⁻¹ s⁻¹) |
| *kₐ* | = association rate constant (m⁻¹ s⁻¹) |
| *k_{d}* | = dissociation rate constant (s⁻¹). |
| *kₘ* | can be a fitted parameter or alternatively can be estimated from the following equation (assuming that the use of dextran hydrogel does not contribute to mass transport limitations): |
| ${k}_{m} = {10}^{9} . {M}_{r} .1.43. [ \frac{1 - {( \frac{{L}_{1}}{{L}_{2}} )}^{2 / 3}}{1 - ( \frac{{L}_{1}}{{L}_{2}} )} ] . \sqrt[3]{\frac{{D}^{2} . F}{{H}^{2} . W . {L}_{2}}}$ | |

where

| | |
|---|---|
| *F* | = flow rate (m³ s⁻¹) |
| *L₁* and *L₂* | = lengths (m) of the functionalized surface relative to the start and end of the sensing region, respectively |
| *H* | = height (m) of the flow cell conduit |
| *W* | = width (m) of the flow cell conduit. |

Under preferred conditions, the geometry of the flow cell is well defined, allowing these parameters to be held constant. The analyte molecular weight (*Mᵣ*) is usually known or alternatively it can be estimated from *D.*

In one aspect of the current invention, a dispersion term is incorporated into the binding interaction model. In the pulse TDi embodiment, it is appropriate to represent *Cᵢₙ* in the binding interaction model with the dispersion term provided in Equation 1. In the sigmoidal TDi embodiment, it is appropriate to represent *Cᵢₙ* in the binding interaction model with the dispersion term provided in Equations 3 and 4. In either embodiment, *Cᵢₙ* is specified as a function of time. Thus, *kₐ, k_{d}, Rₘₐₓ, kₘ,* and *D* (Equation 2) are fitted parameters. If multiple analyte species exist in the injected sample, then the appropriate dispersion term is included as a separate term for each dispersed species. In many cases, mass transport limitation is negligible, where *Cᵢₙ* = *C*, and the two-compartment 1:1 kinetic interaction model reduces to the simple "rapid mixing" model by eliminating Equation 5 and is commonly referred to as 1:1 pseudo-first-order kinetic interaction model. These kinetic models have many variations that account for deviations from the assumption of a 1:1 interaction. For example a ligand with more than one analyte binding site requires a heterogeneous analyte kinetic interaction model. If the analyte possesses more than one binding site for the ligand then a heterogeneous ligand interaction model can be applied. If the formation of binding complexes proceed through the formation of a short lived intermediate complex, then a kinetic interaction with conformational change model can be chosen. If a large population of different analyte species co-exist, where each possesses different binding properties to the immobilized ligand, then an analyte distribution model can be chosen. Similarly, a distribution model can be applied when a large population of ligand species exist (i.e. ligand distribution model). The distribution model assumes that a large population of analyte species, or ligand species, co-exist and distribution analysis methods are applied in the fit to generate a probability distribution of the affinity space (i.e. plot of *kₐ* versus *K_{D}, k_{d}* versus *K_{D}*) constructed from fitting a set of binding interaction curves. Each of the above models can be simplified when the influence of kinetic rate constants is minimal thereby reducing each to a simple affinity analysis.

Using the current method, any of the above interaction models of interest can be appropriately modified to incorporate a dispersion term. For example, a two-site binding interaction model can be chosen where the response recorded by the biosensor (*R₍ₜ₎*) is given by the sum of multiple components as represented by the following: ${R}_{\left(t\right)} = AB + {AB}_{2} + {RI}_{analyte}$ with fitted parameters *kₐ, kₐ₂, k_{d}, k_{d2}, Rₘₐₓ, Rₘₐₓ₂,* and *D, Dₛₒₗᵥₑₙₜ.*

In Equation 8, *AB* and *AB₂* are the response components due to affinity complexes formed between the analyte at two independent analyte binding sites. A similar approach can be used to represent a multisite interaction model where more interaction sites exist. Each binding component can include affinity and/or kinetic parameters as appropriate to the data set to be analyzed. To express each binding component as a kinetic model, a separate set of binding rate equations (Equations 5 and 6) is required for each binding component. *RI_{analyte}* is the bulk refractive index term associated with the analyte and is defined by equation 1 when running pulse TDi or Equations 3-4 when running sigmoidal TDi. This bulk refractive index term is required because refractive index based detector for label-free biosensing measure all contributions to refractive index change and includes surface mass loading, but also bulk refractive index changes. An additional bulk refractive index term for any solvent mismatch may be defined by the appropriate Taylor dispersion equations with the addition of a solvent diffusion coefficient (*Dₛₒₗᵥₑₙₜ*).

For example, if a solvent such as dimethylsulfoxide (DMSO) is present at a slightly different concentration in the sample fluid relative to the analyte-free carrier fluid, then the mismatched DMSO concentration will also undergo Taylor dispersion, and hence, a separate bulk refractive index dispersion term can be included to account for the contribution of this component to the resulting binding response curve.

Experiments performed in a manner that ensures significant mass transport limitation (MTL) can be exploited to determine the injected analyte concentration *Cᵢₙ*. Mass transport limitation is a condition where the binding interaction curves are influenced by the flux of analyte from the bulk liquid through the non-stirred boundary layer at the sensing region. This influence is concentration dependent and hence can be exploited to solve for concentration. For example, *kₘ,* as described in equation 6, is held constant and the two compartment model can be fitted to solve for the interaction constants and *Cᵢₙ*.

Additionally, a multi-site affinity interaction model that assumes a rapid approach to steady-state (i.e. kinetic terms not required) can also be employed. The three-site version of a multisite affinity model can be expressed as: ${R}_{eq} = \left[{R}_{max 1}*C/\left({K}_{D 1}+C\right)\right] + \left[{R}_{max 2}*C/\left({K}_{D 2}+C\right)\right] + \left[{R}_{max 3}*C/\left({K}_{D 3}+C\right)\right]$

The equilibrium response (R_{eq}) is the sum of three affinity binding terms each defined by independent parameter values for the saturation response (Rₘₐₓ) and affinity constant (K*_{D}*). However, this affinity model cannot be fitted directly to the real-time TDi binding response curve. Therefore, a real-time affinity isotherm model is used (Equation 10 below) that is analogous to Equation 9, but where R_{eq} was expressed as a function of injection time (R_{eq(t)}) allowing the isotherm model to be fitted directly to the TDi curve. Fitting Equation 10 directly to the binding response curve enables D to be determined from the fit. ${R}_{eq \left(t\right)} = {R}_{max 1} * {C}_{\left(t\right)} / \left({K}_{D 1}+{C}_{\left(t\right)}\right) + {R}_{max 2} * {C}_{\left(t\right)} / \left({K}_{D 2}+{C}_{\left(t\right)}\right) + {R}_{max 3} * {C}_{\left(t\right)} / \left({K}_{D 3}+{C}_{\left(t\right)}\right)$ where C₍ₜ₎ is given by the appropriate dispersion term (Equation 1 for pulse TDi and Equation 3 and 4 for sigmoidal TDi). Since this affinity model is complex, it is preferable to use two or more TDi curves in order to constrain fitted parameters globally for improved performance. The benefit of global model fitting is the added information content that results from including two binding curves recorded under different conditions. Performing TDi at two different neat concentrations is a simple approach, but does require a second analyte sample of different neat cocnentration be added to the sample rack. To avoid having to prepare analyte dilutions, it is possible to obtain two curves under significantly different conditions by changing the dispersion conditions and not the neat analyte concentration. For example, replicate TDi injections performed at different injection flow rates produce different Taylor dispersion profiles. The two TDi binding response curves are offset with respect to time because 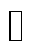 is a function of flow rate, but they are readily fitted with a binding interaction model where one or more parameters are constrained globally. In one aspect, the binding interaction constants and the diffusion coefficient are fitted globally while 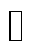 would be fitted locally. This approach can be applied generically to the wide range of binding interaction models available. Furthermore binding interaction models may still be fitted to sub-regions for each curve in the curve set for global model fitting. However, when time is limiting, it is possible to obtain reliable parameter values from fits to single curves providing higher throughput.

There are a variety of additional aspects that can be used in connection with the injection methods described herein. In one aspect, a soluble ligand that interacts with the analyte is added to the injected fluid sample. This causes a fraction of the analyte to be bound in bulk solution and unavailable for binding to the ligand at the sensing surface. The degree to which binding of analyte to the ligand at the sensing surface is reduced is dependent on the affinity of the analyte for the second soluble ligand in solution. This allows competitive models to be used to calculate the affinity or kinetic parameters for solution phase binding events. TDi can be used to generate a continuous dose response in the soluble ligand while holding the analye concentration constant thereby allowing a complete competitive affinity analysis from a single TDi injection. It should be understood that the binding interaction model need not represent any biophysical binding interaction process itself. In fact, a phenomenological binding interaction model that can account for some of the interaction properties can be utilized. Alternatively, an arbitrary mathematical model with a set of parameters that are completely unrelated to the actual interaction parameters can often suffice when kinetic constants and affinity are not required. However, these models may also be adapted to include a dispersion terms. Hence, the dispersion process and the associated diffusion coefficients may be estimated. The arbitrary models can fit to data that cannot be defined by conventional biophysical models, but can act as calibration curves for relating a response to other assay parameters of interest.

In order to illustrate various aspects of the methods described above, the following Examples are provided. It should be understood that various modifications could be applied without departing from the scope of the invention as described in the appended claims. As such, the Examples should not be construed to limit the present invention.

### EXAMPLES

### Instrument, Sensor Chips, and Chemicals

A SensíQ Pioneer instrument (FLIR/ICx-Nomadics, Oklahoma City, OK) was used in the following Examples. All injections were performed at 25 °C unless otherwise stated. All other reagents were obtained from Sigma (St. Louis, MO). The running buffer contained 10 mM Hepes and 150 mM NaCl, pH 7.4 (HBS), with 0.001 % Tween 20 (HBS-T) and 3 mM EDTA (HBS-TE) unless otherwise stated. For all FCI assays, the flow channel conduit was omitted so that the volume from the injector valve to the flow cell was < 3 µL thereby reducing any dispersion such that the sample concentration can be assumed approximately constant over the course for FCI. Recombinant human epidermal growth factor receptor 2/crystallizable fragment (rHErbB2/Fc) chimera was purchased from R&D Systems (Minneapolis, MN) and anti-ErbB2 single chain variable fragment (scFv) was a kind donation by a company that wishes to remain anonymous.

### General TDi Analysis

Ethylene tetrafluoroethylene (ETFE) and polyether ether ketone (PEEK) tubing was obtained from Upchurch Scientific (Oak Harbor, WA) and cut to the appropriate dimensions as described for the dispersion tube in the following Examples. The tubing or flow channel conduit was fitted in the SensíQ Pioneer to connect the injector valve to the reaction flow cell conduit. Five different geometries of the flow channel conduit are included in the Examples: tube A with Vt = 509 µL and d = 0.227 mm; tube B with Vt = 500µL and d = 0.38 mm; tube C with Vt = 512 µL and d = 0.50 mm; tube D with Vₜ = 52, and d = 0.25 mm, and tube E with Vₜ 120 µL and d = 0.25 mm. The autosampler performed unattended loading of samples contained in a cooled aluminum sample rack. Routine purging methods were incorporated into each sample cycle. The system was pre-cleaned with 0.5% sodium hypochlorite and then coated with 0.1% Tyloxapol in water to minimize analyte interactions with the surface of the flow channels. A COOH V sensor chip was installed in the SensíQ Pioneer system and the system was primed with HBS-TE. The flow rate was chosen to give a low (< 600 s) mean retention time, allowing Taylor analysis to be conducted at analysis cycle times comparable to a single FCI cycle. The viscosity of HBS was assumed to be 0.94 Cp at 25 °C and a linear correction factor (CF) for both temperature and viscosity was employed to standardize D measurements recorded under non-standard conditions.

### Curve-Fitting

Curve fitting to kinetic interaction curves was performed using GradFit, a custom simulation and curve-fitting package developed by BioLogic Software, Inc., (Campbell, Australia). GradFit software uses numerical integration of the binding rate equations (Equations 5-6) to generate simulations and fit interaction models to interaction curves. A scripting interface provided a convenient model building environment to construct interaction models with, or without, a mass transport term. Data fitted in GradFit were exported into GraphPad Prism and plotted. Qdat, a customized version of the Scrubber data analysis software from Biologic Software, Inc., (Campbell, Australia) was employed for double referencing of all data sets with the exception of the bulk refractive index-based TDi data, which was referenced by simply subtracting the response for a blank buffer sample. The pulse TDi dispersion term (Equation 1) or sigmoidal TDi dispersion terms (Equations 3 and 4) were included when fitting all TDi data to the described affinity interaction model.

### ZEXAMPLE 1

### Determination of analyte concentrations and affinity constants using the pulse TDi and sigmoidal TDi methods

The purpose of this Example is to demonstrate that pulse TDi and sigmoidal TDi methods can be used to determine affinity constants in a label-free biosensing application. More specifically, warfarin was injected under both pulse TDi and sigmoidal TDi conditions and real-time binding responses to human serum albumin (HSA) were measured and fitted to an affinity interaction model where the analyte (warfarin) concentration was represented by a dispersion term specific for either pulse TDi (Equation 1) or sigmoidal TDi (Equation 3 and 4).

### Methods and Materials

Warfarin was prepared as a 33 mM stock in DMSO and then diluted in HBS-TE to the appropriate injected neat sample concentration. The samples contained approximately 3% DMSO and the resulting bulk refractive index offsets were eliminated by conventional double referencing. The presence of DMSO offsets also cause a non-negligible offset in sensitivity between sensing channels that is related to an excluded volume effect. The sensing channels were normalized using bulk refractive index standards to compensate for this effect.

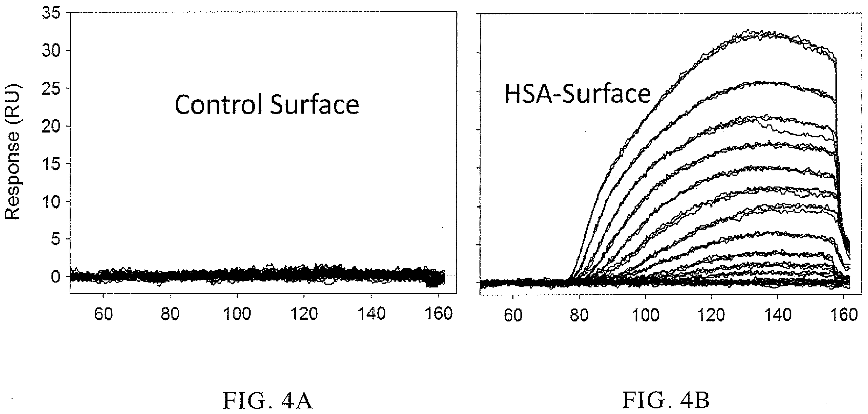

HSA was immobilized by standard amine coupling onto a COOH V chip surface in a flow cell conduit, providing a loaded mass equivalent to 4.5 kRU. The surface was allowed to stabilize for 24 hours to limit conformational shifting of HSA. The same flow cell conduit included a control surface that was not coated with HSA. For pulse TDi's (FIGS. 4A-4D) the flow cell conduit was in fluid communication with a dispersion tube (flow channel conduit) having a total volume capacity of 52 µL and a diameter of 0.25 mm. Pulse TDi was performed by injecting 5 µL of sample at 25 µL min⁻¹ into the dispersion tube for a duration of 158 seconds. These injection conditions were repeated in triplicate for a series of twelve samples, each sample representing a serial 2-fold dilution (0.244 nM to 1 mM) in order to demonstrate the reproducibility of the method. Data was double referenced in Qdat prior to fitting to an affinity interaction model.

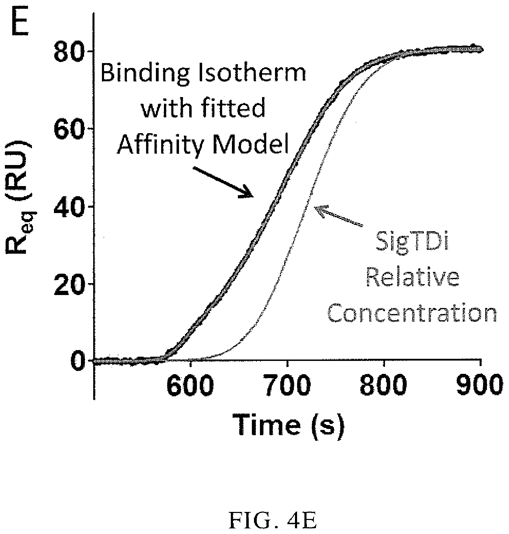

For sigmoidal TDi's (FIG. 4E), the flow cell conduit was in fluid communication with a larger dispersion tube having a total volume capacity of 120 µL and a diameter of 0.25 mm. Sigmoidal TDi was performed by injecting 60 µL of 1 mM warfarin at 10 µL min⁻¹ into the dispersion tube for a duration of 1040 seconds. Regeneration was not necessary as warfarin dissociated rapidly from the HSA-coated surface.

For all sigmoidal TDi injections, rather than injecting a sample volume of approximately 1.5-fold the dispersion tube volume, the sample volume was reduced to 0.5-fold the dispersion tube volume using the following approach. Once the sample volume has entered the dispersion tube, an additional volume of running buffer (i.e. chasing buffer) continues to enter the dispersion tube such that the total volume injected upon completion of the sigmoidal TDi is approximately 1.5-fold the dispersion loop volume. The chasing buffer entering the dispersion loop is prevented from mixing with the leading sample volume by including an air segment (typically 2-5 µL) that separates both liquids. This allows a full sigmoidal TDi profile to be completed using a relatively low sample volume.

### Results and Discussion

Referring now to FIG. 4A, superimposed double referenced response curves elicited from the control sensing surface are shown for all samples (0.244 nM to 1 mM warfarin) injected under the pulse TDi conditions. The effectiveness of double referencing is evident given the absence of a measurable response at the control sensing surface. The responses elicited at the HSA-coated sensing surface are therefore attributed to the interaction between warfarin and HSA and hence, any interference has been accurately subtracted from the binding response curves. Interferences that can contribute to the response include thermal drift of the response and non-reproducible non-specific binding at the ligand-coated and non-coated sensing regions. As used herein, the term "double referencing" refers to the subtraction of two control response curves from the binding response curve recorded over the ligand-coated sensing surface. The first of these control curves is the response curve recorded from the non-coated sensing surface (reference curve) and the second is a response curve recorded for a replicate sample injection where the analyte was omitted from the sample (blank curve). This is a standard referencing procedure in real-time label-free biosensing and applies equally to TDi-based analyses.

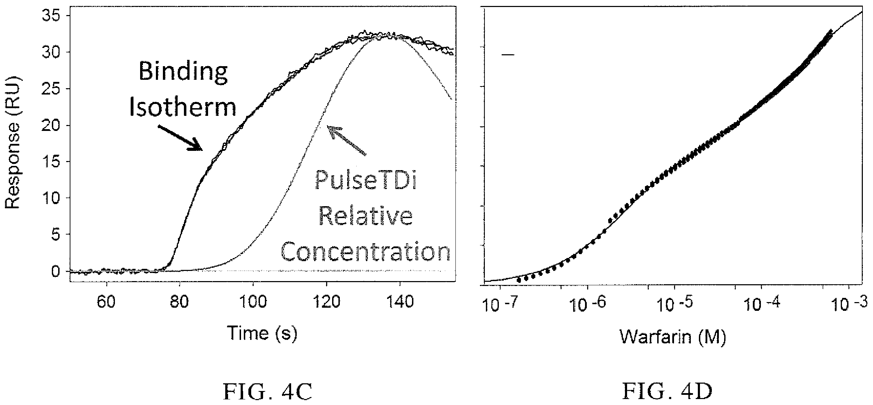

Referring now to FIG. 4B, superimposed double referenced response curves elicited from the HSA-coated sensing surface are shown for all samples (i.e. serial two-fold dilutions from 0.244 nM to 1 mM warfarin) injected under the pulse TDi conditions. Each concentration was performed in triplicate and were virtually superimposable demonstrating a high degree of reproducibility for TDi analysis. A range of concentrations were included in order to demonstrate the expected trend where higher analyte concentrations begin binding earlier than lower analyte concentrations as a result of the gradient in concentration produced by dispersion. FIG. 4C shows the triplicate binding curves for 1 mM warfarin (the top curve set from FIG. 4B) re-plotted with the expected relative warfarin concentration profile calculated from the pulse TDi equation Equations 1-2 where the diffusion coefficient (D) was calculated from the Stokes-Einstein-Sutherland equation. The experimental curves show that warfarin begins binding when the relative concentration is relatively low (< 1/1000th of neat) while the maximum response corresponds to the analyte gradient peak.

Referring now to FIG. 4D, a subset of the response points were selected from the 1 mM warfarin curves (shown in FIG. 4C) and plotted against the expected concentrations for each selected response point. The resulting isotherm was fitted to a two-site affinity model (Equation 9). A two site affinity model was selected since warfarin is known to bind predominantly to two separate affinity binding sites on human serum albumin. The resulting affinity constants generated from the model fitting (*K_{D1}* = 2.70(6) µM; *K_{D2}* = 310(20) µM were in good agreement with literature values. Thus, the use of a dispersion term (Equation 1) in the affinity model to represent the warfarin concentration at any given time point produces accurate affinity constants thereby validating the pulse TDi method.

Referring now to FIG. 4E, binding responses were recorded (in triplicate) for a neat sample concentration of 1 mM warfarin injected under sigmoidal TDi conditions. The binding response curve (leftmost curve) was plotted together with the expected relative warfarin concentration profile (appears shifted to the right) calculated from Equations 3 and 4 (*τ* at 720 seconds) where the diffusion coefficient (*D*) was calculated from the Stokes-Einstein-Sutherland equation. A three-site real-time affinity model (Equation 11) was fitted using GraphPad Prism and the fitted parameters for the two specific binding sites of interest were determined. As stated above, there are only two specific binding sites for warfarin, however, additional non-specific binding to undefined regions occurs when the warfarin concentration is very high. Adding a third binding site to the affinity model compensates for interference Aassociated with the presence of these additional non-specific sites allowing determination of the affinity constants for the two specific sites of interest. The interaction parameters associated with the third site are meaningless because the binding is non-specific. The parameters returned for the two specific sites are as follows for 95% confidence limits: *D* = 5.0 - 5.2 (x 10⁻¹⁰) m² s⁻¹; *Rₘₐₓ₁* = 13.3 - 14.2 RU; *K_{D1}* = 2.4-3.0 (x 10⁻⁶) M; *Rₘₐₓ₂* = 14.9 - 17.6 RU; *K_{D1}* = 6.7-9.0 (x 10⁻⁵) M; ResSD = 0.26 RU. The plot of FIG. 4E shows three curves in total, the relative concentration profile (which appears shifted to the right), the experimental binding response curve, and the fitted model curve which is indistinguishable from the experimental curve due to the excellent fit giving an average residual standard deviation of 0.26 RU. The affinity constants determined by the sigmoidal TDi method are consistent with those determined from the pulse TDi method. Furthermore, the real-time affinity isotherm model defined by Equation 10 was directly fitted to the sigmoidal TDi curve, which includes all data points, as a more convenient alternative to the standard isotherm plot (i.e. R_{eq} v concentration). Fitting directly to the real-time response curve preserves time dependent information that would otherwise be lost and therefore improves the performance of the model fit and enables *D* to be determined. The 95% confidence limits for each returned parameter indicated that both high affinity, and low affinity, sites are well determined from a single TDi curve. However, when assay run times are less critical, it is advisable to include another sigmoidal TDi curve at a 10-fold lower concentration to further improve the parameter confidence intervals by fitting parameters globally to both response curves. This also improves resistance to occasional interferences that do not subtract exactly upon double referencing. It is possible that other more complex binding models may benefit from application of TDi in a similar way.

### EXAMPLE 2

### The use of TDi in affinity ranking

In some applications, it is not appropriate to fit a kinetic model to a binding response curve and a simple means of providing a kinetic/affinity ranking can suffice. For example, many proteins degrade rapidly or shift between different conformational states and, if these changes occur while a kinetic/affinity analysis is being performed, the recorded data represents the average kinetic/affinity state during the assay. Indeed, conformational shifting may be responsible for unstable binding of drugs to ligand immediately after immobilization where a number of affinity states coexist at binding sites. Because the TDi method provides a continuous progression of changing analyte concentration, these changes in binding properties can be effectively monitored thereby providing a more detailed account of the changing binding characteristics. In this Example, the TDi method was used to track changes in the affinity for a two-site kinetic/affinity ranking for the interaction between warfarin and HSA as HSA transitioned rapidly between different transient conformations.

### Materials and Methods

Human serum albumin (HSA) was immobilized by standard amine coupling onto a COOH5 chip surface, giving a loaded mass equivalent to 10 kRU. Warfarin was prepared as 33 mM stocks in dimethyl sulfoxide (DMSO) and then diluted to a neat working concentration of 3 mM in HBS-TE with a final DMSO concentration of 3%. Sigmoidal TDi's (15 replicate injections) were performed at 15 minute intervals by injecting 250 µL of 3 mM warfarin at a rate of 100 µL min⁻¹ through a dispersion tube (flow channel conduit) having a total volume of 509 µL and a diameter of 0.227 mm for a contact time of 390 seconds. Once the sample volume has entered the dispersion tube an additional volume of running buffer continues to enter the dispersion tube such that the total volume injected upon completion of the sigmoidal TDi is approximately 1.5-fold the dispersion loop volume. This is necessary in order to produce a complete sigmoidal dispersion gradient within the flow cell conduit. The "chasing" buffer entering the dispersion loop is prevented from mixing with the leading sample volume by including an air segment (typically 2-5 µL) that separates both liquids. This allows a full sigmoidal TDi profile to be completed using a relatively low sample volume. Data was recorded at 8 Hz for enhanced resolution of the derivative curves.

### Results and Discussion

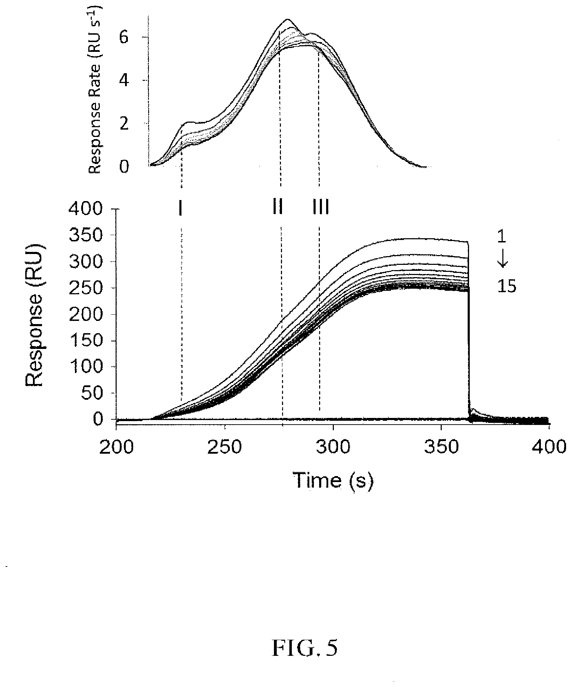

Referring now to FIG. 5, a time progression series of overlaid sigmoidal TDi binding response curves for a warfarin-HSA interaction is provided. The inset represents a derivative plot of the first seven binding curves after a five-point smoothing. As demonstrated in FIG. 5, the derivative curves contain three or more distinct peaks associated with different binding site populations and indicated by the vertical broken lines and Roman numerals. The progression of these binding sites towards lower affinity (i.e. shift to right) is evident. Therefore, the overlaid binding response curves in FIG 5 effectively represent a time series progression where dynamic changes in affinity can be qualitatively compared.

### EXAMPLE 3

### Bulk Refractive Index TDi for Estimation of Diffusion Coefficient (D)

The purpose of this example is to demonstrate that TDi provides an estimate of diffusion coefficients that are in agreement with those established in the literature for a group of analytes. For experimental determination of diffusion coefficients, the concentration of analyte should be high enough to produce a significant bulk refractive index response. A series of biomolecular standards was prepared at 10 mg/mL in HBS-TE running buffer immediately before priming the system into the same buffer to ensure that the bulk refractive index difference between the injected sample and the continuous flow buffer was due entirely to the added biomolecule. The series of biomolecular standards tested included the following: glycine (*Mᵣ* 75), alanine (*Mᵣ* 89); β-cyclodextrin (*Mᵣ* 1135); vancomycin (*Mᵣ* 1485); polyvinyl pyrrolidine (*Mᵣ* 1.0 x 10⁴); ribonuclease (*Mᵣ* 1.37 x 10⁴); carbonic anhydrase II (*Mᵣ* 3.0 x 10⁴); ovalbumin (*Mᵣ* 4.5 x 10⁴); bovine serum albumin (BSA) (*Mᵣ* 6.6 x 10⁴); a single chain Fv antibody fragment (*Mᵣ* 2.75 x 10⁴); mouse IgG₁ (*Mᵣ* 1.5 x 10⁵); and dextran (*Mᵣ* 1.07 x 10⁴, 3.7 x 10⁴, 5.0 x 10⁵).

The pulse TDi method was used where a sample volume of 12 µL was loaded and injected at 50 µL min⁻¹ followed by running buffer and allowed to migrate through a dispersion tube (flow channel conduit) having a total volume capacity of 509 µL and a diameter of 0.227 mm (i.e. tube A) before reaching the sensing regions. The bulk refractive index was recorded where one response unit (RU) was equivalent to 1 µg/mL analyte. The recorded bulk refractive index response curves were fitted to Equation 1 using GraphPad Prism (GraphPad Software, Inc., La Jolla, CA). In bulk Pulse TDi analysis, *Cₜ* was replaced by RI_{analyte}(t) and Cᵢₙ was replaced by RIₘₐₓ. in Equation 1. The injected sample volume, volume of the tube, and *D* were fitted locally for each curve while the remaining parameters were held constant. The recorded bulk refractive index response did not possess a surface binding component. The injection was repeated in quadruplicate and curves were superimposable for a given analyte. The volume of sample injected was confirmed by integrating the peak. In each case, the target volume of 12 µL was attained to within 5%. The procedure was repeated for all analytes listed above.

### Results and Discussion

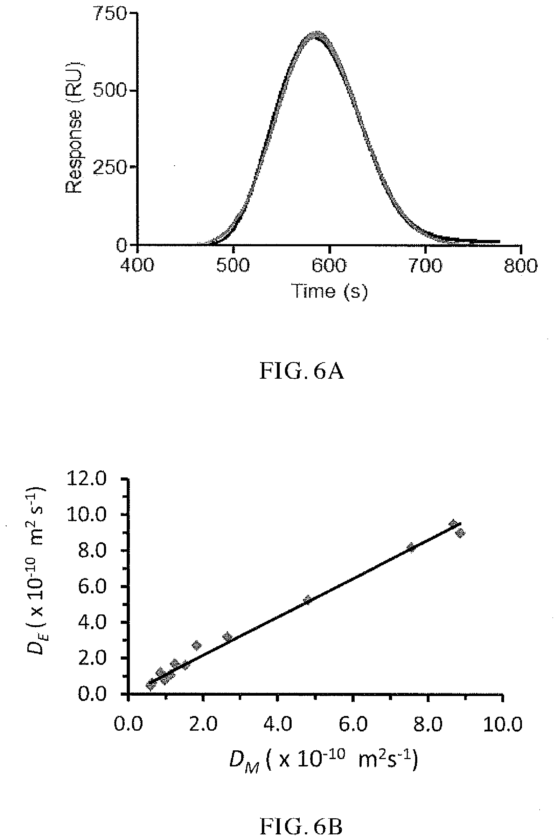

Referring now to FIG. 6A, a set of four superimposed curves for vancomycin are shown with the model fitted. The measured bulk refractive index (black curves) was fitted (red curve) with Equation 1 where the geometry of the tube and flow rate were held constant, allowing accurate estimation of the diffusion coefficient. As demonstrated in FIG. 6A, the measured response curve and theoretical/fitted curve are almost superimposable (R²=0.999) indicating that the model is appropriate and the experimental interferences are acceptably low.

The diffusion coefficients for the other biomolecules listed above were determined by the same method as described for vancomycin and the measured diffusion coefficients (*D*_{measured} - X-coordinate) of each analyte were compared to the expected literature values (*D*_{expected} - Y-coordinate). A fitted linear regression retuned a linear relationship give by, *D*_{expected} = 1.08 ^{∗} *D*_{measured}, as shown in FIG. 6B. This agreement between the TDi curves and theory validates the application of the Taylor dispersion model. The determination of diffusion coefficients by solution phase Taylor dispersion methods provides a new practical application for biosensing experiments. In particular, *D* can also be determined as a fitted parameter from the solid-phase binding interaction curves, reducing the amount of analyte sample required and allowing crude analyte samples to be used. Measurement of the diffusion coefficient by either solution phase (i.e. bulk refractive index response) or solid phase (i.e. specific binding response) TDi method is also useful in confirming the absence of analyte aggregates.

Analyte aggregation is common for most classes of analyte and these binding events do not obey a 1:1 interaction model, therefore require more complex models. However, the application of such multivalent models remains controversial and it is more desirable to control environmental conditions to prevent aggregation. Screening for suitable conditions is difficult as other sources of complexity, such as the presence of multiple binding sites on a single analyte molecule, may be present. TDi enables unambiguous determination of the apparent diffusion coefficient which in turn enables the apparent molecular weight to be determined. Analyte aggregation gives rise to apparent molecular weights that are significantly greater than the expected "true" molecular weight of the analyte thereby identifying aggregation.

### EXAMPLE 4

### A comparison of scFv - ErbB2/Fc interaction analysis using the FCI and TDi methods

### Materials and Methods

For the standard FCI format, a sensing chip with a carboxylated dextran-based hydrogel (COOH V) was installed and the biosensor was primed into HBS-TE buffer. Protein G was immobilized onto two sensing channels by standard amine coupling to a mass equivalent to 1200 RU. Each binding interaction cycle was performed as follows: The fusion protein, ErbB2/Fc, was diluted to 10 nM in running buffer and captured onto the first channel by injecting 50 µL at 25 µL min⁻¹ while the second channel was left uncoated and used for double referencing of the data set. scFv diluted in running buffer was then injected over both channels at 50 µL min⁻¹. Dissociation was observed for 10 min before regenerating (i.e. removal of all non-covalently bound protein) with a 60 second exposure of both channels to 20 mM NaOH. This series of three injections was repeated for each serial 10-fold dilution of scFv from 1000 nM to 0.1 nM. For this standard FCI, the sample does not flow through a dispersion tube but passes immediately from the injector value into the flow cell with a minimum of dead volume (i.e. < 3 µL) in the connector channel.

A sigmoidal TDi was performed by injecting 250 µL of analyte sample at 30 µL min⁻¹ through a dispersion tube (tube B) having a total volume capacity of 500 µL and a diameter of 0.38 mm prior to contacting the sensing surface. As stated earlier, the sample volume is followed by an air bubble and then running buffer giving a total injected volume of -1.5-fold the loop volume in order to yield a complete sigmoidal gradient profile. Triplicate 10-fold dilutions ranging from 1000 nM to 0.1 nM were prepared and injected. The residence time (τ) was equal to the dispersion tube volume divided by the flow rate, and the full sample contact time exceeded τ in order for the gradient to approach the maximum analyte concentration. Therefore, the contact time was chosen such that the total volume injected was approximately 1.5-fold the total dispersion tube volume. Both FCI and TDi assays were performed during the same assay run, over the same sensing surfaces, and using the same reagent preparations in order to minimize experimental variability and enable reliable comparison of the techniques.

### Results and Discussion

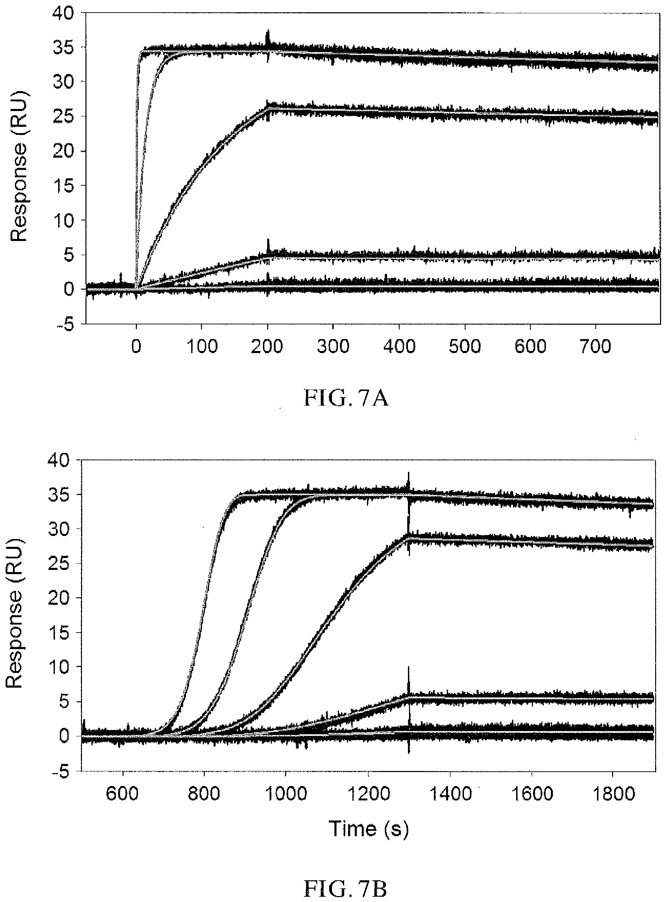

The interaction of scFv with ErbB2-Fc bound to a sensing surface was analyzed by both FCI and sigmoidal TDi giving the response curves in FIGS. 7A and 7B, respectively. FIGS. 7A and 7B demonstrated the fitted model (smooth red curves) superimposed on the experimental curves (rough black curves) for FCI and sigmoidal TDi, respectively. The data acquisition rate was 8 Hz and the SD of the residuals for all fits was < 0.20 RU.

Table 1 shows the model parameter values determined from six separate fittings to the FIG 7B. Each fit is a global fit of the interaction parameters to the curve set in FIG 7B, but the fitting conditions are changed in order to explore the performance of the model when making different assumptions about the model and the data. For example, the first model is the simple model describing a single interaction site obeying 1:1 pseudo-first-order interaction kinetics. In the first global fit of this model to the data (Table 1, Row 1), *D* and concentration are assumed to be known *a priori* and therefore, the model need not solve for these parameters. Decreasing the number of model parameters decreases model complexity and generally improves the performance of the model fit.

**TABLE 1**

| **Model Type** | ***kₐ* (M⁻¹ s⁻¹)** | ***k_{d}* (s⁻¹)** | ***Rₘₐₓ* (RU)** | ***D* (m²s-¹)** | ***kₘ* (RU M⁻¹ s¹)** | ***Conc.* (M)** |
|---|---|---|---|---|---|---|
| **Simple** | 7.094(3) x 10⁵ | 7.20(2) x 10⁻⁵ | 34.597(7) | Fixed | - | Fixed |
| **Simple** | 6.398(1) x 10⁵ | 6.87(2) x 10⁻⁵ | 35.018(2) | 8.878(2) x 10⁻¹¹ | - | Fixed |
| **Simple** | 6.5(2) x 10⁵ | 6.91(2) x 10⁻⁵ | 35.012(2) | Fixed | - | 1.01 µM |
| **Two-Compartment** | 7.15(1) x 10⁶ | 8.90(4) x 10⁻⁵ | 34.531(4) | Fixed | 1.0(1) x 10⁹ | Fixed |
| **Two-Compartment** | 6.592(2) x 10⁵ | 6.94(2) x 10⁻⁵ | 35.007(2) | 8.859(2) x 10⁻¹¹ | Linked to D | Fixed |
| **Two-Compartment** | 6.517(3)) x 10⁵ | 6.92(1) x 10⁻⁵ | 35.012(2) | 8.864(1) x 10⁻¹¹ | 6.9(2) x 10⁸ | Fixed |

Table 1 provides the parameter values returned for global fitting of the simple "rapid mixing" model and the two-compartment model. The data from Figure 7 was fitted with a number of interaction models. Parameters that were constrained to known best fit values are indicated by "Fixed". By definition. *kₘ* is absent from the simple model (Rows 1-3). By representing *kₘ* using Equation [7] and coupling the fitted parameter *D* to this expression it was possible to link the estimation of *kₘ* to estimates of *D* as indicated by "Linked to D". The number in parentheses for each parameter value is the SE in the last significant digit.

Table 1, Rows 2-3 are replicates of the global fit in Row 1, except that one of the fixed parameters is no longer held constant, but instead is fitted along with the usual binding constants. By comparing the binding interaction parameter values for each of these three model fits, excellent agreement is found indicating that the model fit is not overly sensitive to the addition of more model parameters to be solved, and hence, it is not necessary to know these *a priori.* This can be very useful in applications where analyte concentration or molecular weight are unknown and can be determined without sacrificing confidence in the binding constants. Table 1, Rows 4-6 provide a similar analysis for fitting of the two compartment model. In a two compartment model, it is assumed that an analyte concentration gradient exits at the sensing surface and must be accounted for using a mass transport term *km.* In the simple model, *kₘ* is not present (or effectively infinite) implying that a gradient is assumed not to exist. In Row 6, it is noted that both *D* and *kₘ* are accurately returned along with the kinetic constants. In Row 5, it is assumed that *kₘ* can be described by Equation 7, and hence, becomes dependent on analyte *D.* However analyte *D* is part of the TDi dispersion term included in the two compartment interaction model and therefore, we simply assume that analyte *D* of the TDi term (i.e. bulk solution phase diffusion coefficient) is approximately equal to the analyte diffusion coefficient within the diffusion boundary layer which is a component of equation 7 defining *kₘ* the mass transport constant through the non-stirred boundary layer. This change in the model improves the model performance because it eliminates *kₘ* from the model and therefore, lowers parameter correlations and widens the kinetic range of experimental data that can be modeled. In particular, faster interactions defined by high association rate constants become more readily modeled.

In general, characterizing PEGylated antibody fragments requires separate assays to determine the active concentration, *Mᵣ* and kinetic interaction constants. In this Example, we demonstrate that TDi can be used to obtain all three measurements in a single assay. For example, values of *D* recovered from a fit of an interaction model to TDi data can be used to estimate *Mᵣ* from empirical correlations obviating the need for a pure analyte sample at high concentrations as required in techniques such as dynamic light scattering. Experimental conditions can be chosen such that sufficient mass transport limitation (MTL) is present for estimation of the active analyte concentration while also estimating kinetic interaction constants and *D* from the fitted model. The data in FIG. 7 was recorded on a low MTL surface and, intuitively, analysis of this data would not be expected to provide accurate estimation of parameters that depend on MTL such as active concentration. Surprisingly, however, it was possible to obtain good estimates for *kₐ, k_{d}, D, kₘ,* and active concentration from this data set. The degree of MTL can be estimated as follows: %MTL = 100^{∗}(*kₐ*^{∗}Rₘₐₓ)/[(*ka*^{∗}Rₘₐₓ)+ *kₘ*], which gives a value of 3.1% for the TDi data in FIG. 7B.

A global fit of a 1:1 interaction model to FCI data gave kinetic constants that were comparable (< 15% variation in any fitted parameter) to those obtained from the sigmoidal TDi model fit, and the average SD of the residuals was low (∼ 0.2 RU) for both data sets. All replicates were superimposable and sigmoidal TDi data was comparable in quality to FCI data. The absence of any systematic deviation of the fitted model over such a wide concentration range (four orders of magnitude) implies that the sigmoidal TDi concentration profile conforms to model predictions even at high dilution factors. The fitted TDi model returned *D* = 8.878(2) x 10⁻¹¹ m² s⁻¹ for the scFv analyte, which was within 5% of the expected value. In addition, the fitted *kₘ* from TDi was in excellent agreement with *kₘ* calculated from theory while *kₘ* from FCI was 50% higher. In general the TDi method provided higher confidence in the measured parameters as indicated by lower SE in estimated parameters.

A particularly useful benefit of TDi becomes apparent when the data in FIG. 7 is fitted locally. The results for fitting both FCI and Sigmoidal TDi data are summarized in Table 2. Table 2 provides *kₐ* values returned from local fitting of TDi curves and FCI curves and relative error with respect to the global parameter values. The data in Figure 7 was fitted with the simple model where *kₐ, k_{d}, Rₘₐₓ,* and *D* were fitted locally to each curve. *kₘ* was constrained to a constant value. The average standard deviation of the residuals for each local fit was < 0.2 RU.

**TABLE 2**

| | FCI | | TDi | |
|---|---|---|---|---|
| nM | *kₐ* (M⁻¹s⁻¹) x 10⁵ | %Relative Error | *kₐ* (M⁻¹s⁻¹) x 10⁵ | %Relative Error |
| 10 | 8.29(3) | 16.86 | 6.03(1) | 5.23 |
| 10 | 6.80(1) | 4.14 | 6.42(1) | 0.90 |
| 10 | - | - | 6.30(1) | 0.99 |
| 100 | 4.59(2) | 35.30 | 6.277(5) | 1.35 |
| 100 | 5.74(3) | 19.09 | 6.426(5) | 0.99 |
| 100 | - | - | 6.358(5) | 0.08 |
| 1000 | 6.62(1) | 6.68 | 5.899(6) | 7.28 |
| 1000 | 8.04(3) | 13.34 | 5.946(6) | 6.65 |
| 1000 | - | - | 5.883(6) | 5.23 |

Local model fitting to a single binding curve far from the *Rₘₐₓ* generally yields unreliable results and therefore such curves were omitted. The difference between TDi and FCI largely concerns determination of *kₐ* with no significant change in determination of *k_{d}.* Fortunately *k_{d}* can be established accurately for single curves that approach *Rₘₐₓ,* a condition that is usually not difficult to achieve. Table 2 provides *kₐ* values returned from local fitting to both data sets in FIG 7 and the relative error with respect to the global parameter values. The data in Table 2 shows that TDi dramatically outperformed FCI giving a 5-fold lower maximum relative error. The SE returned for each parameter was also significantly lower for TDi giving estimates that were lower than those from a global fit to FCI data. The estimates of *D* returned from local fitting were extremely precise giving a CV of only 0.5% and the mean for the set of local fits was within 3% of the global value. Therefore the data strongly suggest that a local fit to a single TDi curve provides kinetic constants that are as robust as those from global fitting to a set of standard FCI curves.

### EXAMPLE 5

### A comparison of furosemide-carbonic anhydrase II interaction analysis using the FCI and sigmoidal TDi methods

### Materials and Methods

Carbonic anhydrase II was immobilized onto a sensing surface by amine coupling with a mass equivalent to approximately 4500 RU. A second sensing surface remained uncoated to provide an accurate reference surface. For FCI, samples were prepared by dissolving furosemide directly in HBS-T to a concentration of 1 mM and then preparing 3-fold dilutions from 0.46 µM to 111 µM from this stock solution. FCI was performed by separately injecting 50µL of each dilution at a rate of 50 µL min⁻¹. EDTA was excluded from the buffer to maintain the binding activity of the Zn-dependent carbonic anhydrase II.

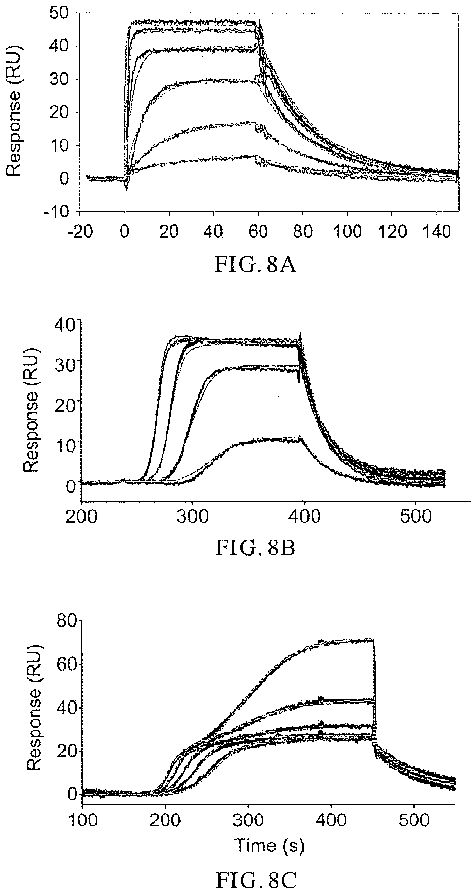

Two separate sigmoidal TDi assays were performed. The first sigmoidal TDi assay (FIG. 8B) was performed by injecting 250 µL of analyte sample at a flow rate of 100 µL min⁻¹ using a dispersion tube having a total volume capacity of 512 µL and a diameter of 0.50 mm. The analyte samples tested comprised four serial 10-fold dilutions of furosemide from 1 µM to 1 mM. A complex binding data set was recorded by allowing the carbonic anhydrase II-coated surface to degrade at 25 °C for 4 days before performing the sigmoid TDi assay. The second sigmoidal TDi assay (FIG. 8C) was performed by injecting 250 µL of analyte sample at a flow rate of 50 µL min⁻¹ using a dispersion tube having a total volume capacity of 512 µL and a diameter of 0.50 mm. The analyte samples tested comprised serial 3-fold dilutions of furosemide from 12 µM to 1 mM. The carbonic anhydrase II-coated surface was permitted to degrade at 25 °C for several days before performing the sigmoid TDi analysis. The second TDi assay was performed at 10 °C to preserve weak non-specific binding in order to mimic a realistic complex data set.

### Results and Discussion

Furosemide is a carbonic anhydrase inhibitor with a *Mᵣ* of 336 and with an expected diffusion coefficient of 5.33 x 10⁻¹⁰ m² s⁻¹ as determined from application of the Stokes-Einstein-Sutherland equation. Kinetic analysis of furosemide binding to immobilized carbonic anhydrase II is shown in FIG. 8A-C. A series of serial 3-fold dilutions were analyzed in triplicate by FCI and the data globally fitted with a 1:1 interaction model as shown in FIG. 8A. As expected, the model interaction was well described by the simple model (Equations 3-6 assuming an infinite *kₘ* (i.e. rapid mixing)) and yielded the following constants: *kₐ* = 1.91(2) x 10⁻⁴ M⁻¹s⁻¹; *k_{d}* = 0.0465(5) s⁻¹; ResSD = 1.2 RU. The analysis was repeated for the first sigmoidal TDi assay and the results for a fit to a global 1:1 interaction model are shown in FIG. 8B. Replicate TDi curves showed high reproducibility at each injected concentration and the kinetic constants were comparable with those from FCI analysis and are as follows: *kₐ* = 2.232(1) x 10⁻⁴ M⁻¹s⁻¹; *k_{d}* = 0.0485(5) s⁻¹; D = 5.36(1) x 10⁻¹⁰ m²s⁻¹; ResSD = 0.91 RU. The TDi curves covered a concentration range of 1000-fold while FCI curves covered a much lower range of 252-fold. The FCI concentrations approximate the limits that produce sufficient curvature for reliable kinetic analysis. Therefore, concentrations outside this range provide little kinetic information. In contrast, TDi curves provide adequate kinetic curvature over a wider range being effectively unbounded at higher concentrations. The fitted parameter, *D,* was practically identical to the predicted value indicating that the analyte was not aggregated. Both TDi and FCI gave reasonable SD of the residuals for each global fit. The SE associated with fitting *kₐ* to TDi data was 20-fold lower than that from FCI data, indicating higher confidence in TDi analysis.

To investigate resolution of two-site binding by TDi, a carbonic anhydrase II-coated sensing surface was allowed to degrade and binding of furosemide was recorded at 10 °C, as shown in FIG. 8C. Non-specific binding (NSB) became significant at higher analyte concentrations presumably because the degraded carbonic anhydrase II presented more hydrophobic pockets than the native form of the protein. Visual inspection of the data from the degraded carbonic anhydrase II surface revealed that specific analyte binding occurred earlier for the higher affinity site, enabling the curve fitting algorithm to readily distinguish both components. A global fit of a two-site interaction model to the TDi data resolved the kinetic constants for the specific affinity site in the presence of a larger response from the NSB site while also returning a *D* of 4.49(1) x 10⁻¹⁰ m²s⁻¹. The values of the kinetic constants are as follows: *kₐ* = 1.89(2) x 10⁻⁴ M⁻¹s⁻¹; *k_{d}* = 0.01524(4)5 s⁻¹; ResSD = 1.0 RU. Low variation (3.6% coefficient of variation) in *kₐ* between curves was observed when each curve was fitted separately (i.e. locally) indicating that a single curve provided a reliable estimate of the kinetic constants. More significantly, NSB comprising -70% of the total binding response did not interfere significantly with estimation of the kinetic constants from single TDi curves. The value of *D* obtained from the two-site fit was within 10% of the expected value, after correction for a temperature offset of 15 °C, suggesting that analyte aggregation at the lower analysis temperature was not significant.

Importantly, if the weak non-specific binding response was not well behaved, but instead was caused by analyte aggregates, then it would not be possible to model this region of the curve. In addition, the dissociation phase would also suffer from distortions making analysis of the whole curve impossible. However, since high affinity sites are occupied before non-specific sites it may sometimes be possible to obtain approximate values for binding interaction constants for the high affinity site by confining the fit to that region of the curve. In cases where *k_{d}* is relatively fast (i.e. drug discovery applications), then there is usually sufficient information in this region to specific both the *kₐ* and *k_{d}*. However, a simple affinity model as described in Equation 9 can be fitted to a sub region of the curve(s) where a rapid approach to a steady state can be assumed (as is the case in FIG. 8C). The number of binding site terms can be chosen appropriately to match the sub region being fitted but a two site model is usually sufficient.

In the kinetic and affinity models, typically, the entire data set (i.e. during the binding phase and during the dissociation phase except for where steady state is presumed ("real-time isotherm") is fitted to the model. However the TDi method allows model fitting to a small, defined region of a given binding curve. For example, referring to FIG. 8C, and specifically to the first curve, it consists of an early rise in response elicited by binding to a high affinity binding site followed later by a binding response to low affinity sites. If a kinetic or affinity model is fitted and confined to the early region before the weak site begins binding, then reasonable values can be obtained for the kinetic constants of the high affinity site of interest. In many cases the weak site is of no value so this analysis avoids having to work with very complex events that only happen in practice. This approach is particularly valuable if the weak binding turns out to be non-specific and binds everywhere (surface and ligand) uncontrollably. In this situation, the dissociation phase is often badly distorted and so is the weak affinity binding phase. Hence, the entire curve is discarded. Unfortunately, a compound is likely to often exhibit this behavior and must be eliminated from analysis, which is not desirable. However by fitting to a subset of the data confined to the region of interest, it is possible to determine approximate kinetic constants for the site of interest. Under this analysis, the expected analyte *D* may be constrained to a constant from knowledge of its *M_{w}* or it may work without it.

Thus, the present invention is well adapted to carry out the objects and attain the ends and advantages mentioned above as well as those inherent therein. While preferred embodiments of the method has been described for the purpose of this disclosure, changes in the particular types of dispersions created and the associated dispersion terms can be made by those skilled in the art, within the scope of this invention as defined by the appended claims. As used in the appended claims, the terms "Equation 1", "Equation 2", "Equation 3", "Equation 4", "Equation 5", "Equation 6", "Equation 7", "Equation 8", "Equation 9", and "Equation 10" are defined by the corresponding equations provided in the specification.

## Claims

1. A sample injection method for determining the affinity interaction parameters of analyte-ligand interactions in a biosensor, the biosensor comprising a flow channel conduit (14) in fluid communication with a flow cell conduit (16), said flow cell conduit (16) housing at least one sensing region (18), said sensing region (18) having at least one ligand (19) immobilized thereon, the method comprising the steps of:
obtaining a fluid sample containing an analyte having a starting concentration;
using a single injection passing the fluid sample through the flow channel conduit (14) to the flow cell conduit (16) under either pulse Taylor dispersion injection conditions or sigmoidal Taylor dispersion injection conditions,
wherein under pulse Taylor dispersion injection conditions, the fluid sample has a volume that is equal or less than 10% of the total capacity of the flow channel conduit (14), and
wherein under sigmoidal Taylor dispersion injection conditions, the fluid sample has a volume that is 50% to 100% of the total capacity of the flow channel conduit (14):
wherein said dispersion conditions result in the formation of an analyte concentration gradient having a maximum analyte concentration and a minimum analyte concentration that differ by at least one order of magnitude, and wherein the analyte concentration in the analyte concentration gradient is continuously changing between the maximum analyte concentration and minimum analyte concentration;
measuring the responses elicited by the analyte interacting with the ligand (19) at the sensing region (18) as the analyte concentration gradient progresses through the flow cell conduit (16), wherein the measured responses provide a binding response curve;
selecting a dispersion term that represents the analyte concentration present at the sensing region (18) at any time point during the injection, wherein under pulse Taylor dispersion conditions the dispersion term is represented by Equation 1 $C \left(t\right) = \frac{2 {C}_{in} {V}_{i}}{{π}^{3 / 2} {d}^{2} \sqrt{kt}} exp \left[-\frac{0.25 {L}^{2} {\left(1-t/τ\right)}^{2}}{kt}\right]$ where
| | |
|---|---|
| *C(t)* | = analyte concentration at detector (mol m⁻³) |
| *Cᵢₙ* | = concentration of analyte injected (mol m⁻³) |
| *Vᵢ* | = sample injection volumne (m³) |
| *d* | = capillary (flow channel conduit) diameter (m) |
| *τ* | = mean analyte residence time (s) or *L*/*u* |
| *L* | = length of capillary (m) |
| *u* | = average velocity of fluid (m s⁻¹) |
| *k* | = Taylor-Aris dispersion coefficient (m² s⁻¹) which is represented by the following equation: |
| $α \frac{{u}^{2} {d}^{2}}{192 D} + D$ | |
where
| | |
|---|---|
| *D* | = analyte diffusion coefficient (m² s⁻¹) and |
| *α* | = conduit shape factor |
and wherein under sigmoidal Taylor dispersion conditions the dispersion term is represented by Equation 3 $\begin{matrix}C \left(t\right) = \frac{{C}_{in}}{2} \left[1-erf\frac{1 - \frac{t}{τ}}{2 \sqrt{\frac{k}{uL} \frac{t}{τ}}}\right] & \left(t<τ\right)\end{matrix}$
for the time points prior to the time required to inject a volume of fluid sample that is equal to the total capacity volume of the flow channel conduit (14) and Equation 4 $\begin{matrix}C \left(t\right) = \frac{{C}_{in}}{2} \left[1+erf\frac{1 - \frac{t}{τ}}{2 \sqrt{\frac{k}{uL} \frac{t}{τ}}}\right] & \left(t>τ\right)\end{matrix}$ where
| | |
|---|---|
| *C(t)* | = analyte concentration at detector (mol m⁻³) |
| *Cᵢₙ* | = concentration of analyte injected (mol m⁻³) |
| *τ* | = mean analyte residence time (s) or *L*/*u* |
| *L* | = length of capillary (m) |
| *u* | = average velocity of fluid (m s⁻¹) |
| *k* | = Taylor-Aris dispersion coefficient (m² s⁻¹) (*Equation 2*) |
| erf | = Gauss error function |
for time points following the time required to inject a volume of fluid sample that is equal to the total capacity volume of the flow channel conduit (14), wherein Equations 3 and 4 include a dispersion coefficient represented by Equation 2;
incorporating the dispersion term into a binding interaction model; and
determining the binding interaction parameters by fitting the binding interaction model to the binding response curve.

2. The sample injection method of claim 1 wherein the binding interaction model is a kinetic interaction model that enables determination of the association rate constant(s) and the dissociation rate constant(s), wherein the binding interaction model is selected from the group consisting of a two-compartment 1:1 pseudo-first-order interaction model, a simple 1:1 pseudo-first-order interaction model, a heterogeneous analyte interaction model, a heterogeneous ligand interaction model, a conformational change interaction model, an analyte distribution model, a ligand distribution model, and a phenomenological binding interaction model.

3. The sample injection method of claim 1 wherein the binding interaction model is an affinity interaction model that enables determination of the affinity constant(s) and is selected from the group consisting of a 1:1 binding interaction model, a heterogeneous analyte interaction model, a heterogeneous ligand interaction model, a conformational change interaction model, an analyte distribution model, a ligand distribution model, and a phenomenological binding interaction model.

4. The sample injection method of claim 1 wherein the dispersion conditions include having the fluid sample flow through the flow channel conduit (14) as a laminar flow.

5. The sample injection method of claim 1 wherein the dispersion conditions include the flow channel conduit (14) having a total volume capacity from about 10 µL to about 2000 µL and a diameter from about 0.05 mm to 1.0 mm.

6. The sample injection method of claim 1, wherein the flow cell conduit (16) further comprises a sensing region (18b) free of immobilized ligand, wherein the method further comprises the steps of:
measuring a bulk refractive index response at the sensing region (18b) free of immobilized ligand elicited by the analyte concentration gradient as it progresses through the flow cell conduit (16), wherein the measured responses provide a bulk refractive index response curve;
wherein the dispersion term includes a dispersion coefficient represented in part by a diffusion coefficient of the analyte; and
determining the diffusion coefficient of the analyte by fitting the bulk refractive index response curve to the dispersion equation.

7. The sample injection method of claim 6, wherein the dispersion term is represented by Equation 1 where the term *C(t)* is replaced by the refractive index of analyte at time *(t)* and the term *Cᵢₙ* is replaced by the maximum bulk refractive index of the analyte, and wherein the dispersion coefficient is represented by Equation 2.

8. The sample injection method of claim 1, wherein the fluid sample contains a soluble ligand.

9. The sample injection method of claim 8, wherein the soluble ligand is the same ligand that is immobilized on at least one of the sensing regions (18).

10. The sample injection method of claim 1, wherein the dispersion conditions include injecting the fluid sample at an upper flow rate limit that is greater than 3.0 as defined in terms of dimensionless time.

## Patentansprüche

1. Probeninjektionsverfahren zum Bestimmen der Affinitätsinteraktionsparameter von Analyt-Ligand-Interaktionen in einem Biosensor, wobei der Biosensor eine Strömungskanalleitung (14) in Fluidverbindung mit einerDurchflusszellenleitung (16) umfasst, wobei in der Durchflusszellenleitung (16) mindestens ein Erfassungsbereich (18) angeordnet ist, wobei der Erfassungsbereich (18) mindestens einen daran immobilisierten Liganden (19) aufweist, wobei das Verfahren die Schritte umfasst:
Erhalten einer Fluidprobe, welche einen Analyten enthält, welcher eine Anfangskonzentration aufweist;
Verwenden einer einzigen Injektion, welche die Fluidprobe durch die Strömungskanalleitung (14) hindurch zu der Durchflusszellenleitung (16) leitet, und zwar entweder unter Puls-Taylor-Dispersions-Injektions-Bedingungen oder unter sigmoidalen Taylor-Dispersions-Injektions- Bedingungen,
wobei unter Puls-Taylor-Dispersions-Injektions-Bedingungen die Fluidprobe ein Volumen aufweist, welches gleich oder kleiner 10 % der Gesamtkapazität der Strömungskanalleitung (14) ist, und
wobei unter sigmoidalen Taylor-Dispersions-Injektions-Bedingungen die Fluidprobe ein Volumen aufweist, welches 50 % bis 100 % der Gesamtkapazität der Strömungskanalleitung (14) beträgt;
wobei die Dispersionsbedingungen in der Bildung eines Analyt-Konzentrationsgradienten resultieren, welcher eine maximale Analyt-Konzentration und eine minimale Analyt-Konzentration aufweist, welche sich um mindestens eine Größenordnung unterscheiden, und wobei sich die Analyt-Konzentration in dem Analyt-Konzentrationsgradienten zwischen der maximalen Analyt-Konzentration und der minimalen Analyt-Konzentration kontinuierlich ändert;
Messen der Antworten, welche durch die Interaktion des Analyten mit dem Liganden (19) an dem Erfassungsbereich (18) hervorgerufen werden, wenn der Analyt-Konzentrationsgradient durch die Durchflusszellenleitung (16) hindurch fortschreitet, wobei die gemessenen Antworten eine Bindungs-Antwortkurve bereitstellen;
Selektieren eines Dispersionsterms, welcher die Analyt-Konzentration repräsentiert, welche zu einem beliebigen Zeitpunkt während der Injektion an dem Erfassungsbereich (18) vorliegt, wobei unter Puls-Taylor-Dispersions-Bedingungen der Dispersionsterm repräsentiert ist durch Gleichung 1 $C \left(t\right) = \frac{2 {C}_{in} {V}_{i}}{{π}^{3 / 2} {d}^{2} \sqrt{kt}} exp \left[-\frac{0.25 {L}^{2} {\left(1-t/τ\right)}^{2}}{kt}\right]$ worin
| | |
|---|---|
| *C(t)* | = Analyt-Konzentration am Detektor (mol m⁻³) |
| *Cᵢₙ* | = injizierte Analyt-Konzentration (mol m⁻³) |
| *Vᵢ* | = Probeninjektionsvolumen (m³) |
| *d* | = Kapillar-(Strömungskanalleitungs-)Durchmesser (m) |
| *τ* | = mittlere Analyt-Verweilzeit (s) oder *L*/*u* |
| *L* | = Kapillarlänge (m) |
| *u* | = durchschnittliche Fluidgeschwindigkeit (m s⁻¹) |
| *k* | = Taylor-Aris-Dispersionskoeffizient (m² s⁻¹), welcher repräsentiert ist durch die folgende Gleichung: |
| $α \frac{{u}^{2} {d}^{2}}{192 D} + D$ | |
worin
| | |
|---|---|
| *D* | = Analyt-Diffusionskoeffizient (m² s⁻¹) und |
| *α* | = Leitungsformfaktor |
und wobei unter sigmoidalen Taylor-Dispersions-Bedingungen der Dispersionsterm repräsentiert ist durch Gleichung 3 $\begin{matrix}C \left(t\right) = \frac{{C}_{in}}{2} \left[1-erf\frac{1 - \frac{t}{τ}}{2 \sqrt{\frac{k}{uL} \frac{t}{τ}}}\right] & \left(t<τ\right)\end{matrix}$
für die Zeitpunkte vor der Zeit, die erforderlich ist, um ein Volumen an Fluidprobe zu injizieren, welches gleich dem Gesamtkapazitätsvolumen der Strömungskanalleitung (14) ist, und Gleichung 4 $\begin{matrix}C \left(t\right) = \frac{{C}_{in}}{2} \left[1+erf\frac{1 - \frac{t}{τ}}{2 \sqrt{\frac{k}{uL} \frac{t}{τ}}}\right] & \left(t>τ\right)\end{matrix}$ worin
| | |
|---|---|
| *C(t)* | = Analyt-Konzentration am Detektor (mol m⁻³) |
| *Cᵢₙ* | = injizierte Analyt-Konzentration (mol m⁻³) |
| *T* | = mittlere Analyt-Verweilzeit (s) oder *L*/*u* |
| *L* | = Kapillarlänge (m) |
| *u* | = durchschnittliche Fluidgeschwindigkeit (m s⁻¹) |
| *k* | = Taylor-Aris-Dispersionskoeffizient (m² s⁻¹) (*Gleichung 2*) |
| erf | = Gauß-Fehlerfunktion |
für Zeitpunkte, die auf die Zeit folgen, welche erforderlich ist, um ein Volumen an Fluidprobe zu injizieren, welches gleich dem Gesamtkapazitätsvolumen der Strömungskanalleitung (14) ist, wobei die Gleichungen 3 und 4 einen Dispersionskoeffizienten umfassen, welcher durch Gleichung 2 repräsentiert ist;
Einbeziehen des Dispersionsterms in ein Bindungs-Interaktionsmodell; und
Bestimmen der Bindungs-Interaktionsparameter durch Anpassen des Bindungs-Interaktionsmodells an die Bindungs-Antwortkurve.

2. Probeninjektionsverfahren nach Anspruch 1, wobei das Bindungs-Interaktionsmodell ein kinetisches Interaktionsmodell ist, welches eine Bestimmung der Assoziationsratenkonstante(n) und der Dissoziationsratenkonstante(n) ermöglicht, wobei das Bindungs-Interaktionsmodell ausgewählt ist aus der Gruppe bestehend aus einem Zwei-Kompartiment-1:1-Interaktionsmodell pseudo-erster Ordnung, einem einfachen 1:1-Interaktionsmodell pseudo-erster Ordnung, einem heterogenen Analyt-Interaktionsmodell, einem heterogenen Ligand-Interaktionsmodell, einem Konformationsänderungs-Interaktionsmodell, einem Analyt-Distributionsmodell, einem Ligand-Distributionsmodell und einem phänomenologischen Bindungs-Interaktionsmodell.

3. Probeninjektionsverfahren nach Anspruch 1, wobei das Bindungs-Interaktionsmodell ein Affinitäts-Interaktionsmodell ist, welches eine Bestimmung der Affinitätskonstante(n) ermöglicht und ausgewählt ist aus der Gruppe bestehend aus einem 1:1-Bindungs-Interaktionsmodell einem heterogenen Analyt-Interaktionsmodell, einem heterogenen Ligand-Interaktionsmodell, einem Konformationsänderungs-Interaktionsmodell, einem Analyt-Distributionsmodell, einem Ligand-Distributionsmodell und einem phänomenologischen Bindungs-Interaktionsmodell.

4. Probeninjektionsverfahren nach Anspruch 1, wobei die Dispersionsbedingungen umfassen, die Fluidprobe die Strömungskanalleitung (14) als eine laminare Strömung durchfließen zu lassen.

5. Probeninjektionsverfahren nach Anspruch 1, wobei die Dispersionsbedingungen umfassen, dass die Strömungskanalleitung (14) eine Gesamtvolumenkapazität von etwa 10 µL bis etwa 2000 µL und einen Durchmesser von etwa 0,05 mm bis 1,0 mm aufweist.

6. Probeninjektionsverfahren nach Anspruch 1, wobei die Durchflusszellenleitung (16) ferner einen Erfassungsbereich (18b) umfasst, welcher frei von immobilisiertem Liganden ist, wobei das Verfahren ferner die Schritte umfasst:
Messen einer Volumen-Brechungsindex-Antwort an dem Erfassungsbereich (18b), welcher frei von immobilisiertem Liganden ist, welche durch den Analyt-Konzentrationsgradienten hervorgerufen wird, wenn dieser durch die Durchflusszellenleitung (16) hindurch fortschreitet, wobei die gemessenen Antworten eine Volumen-Brechungsindex-Antwortkurve bereitstellen;
wobei der Dispersionsterm einen Dispersionskoeffizienten umfasst, welcher zum Teil durch einen Diffusionskoeffizienten des Analyten repräsentiert ist; und
Bestimmen des Diffusionskoeffizienten des Analyten durch Anpassen der Brechungsindex-Antwortkurve an die Dispersionsgleichung.

7. Probeninjektionsverfahren nach Anspruch 6, wobei der Dispersionsterm durch Gleichung 1 repräsentiert ist, wobei der Term *C(t)* durch den Brechungsindex des Analyten zur Zeit *(t)* ersetzt ist und der Term *Cᵢₙ* durch den maximalen Volumenbrechungsindex des Analyten ersetzt ist und wobei der Dispersionskoeffizient durch Gleichung 2 repräsentiert ist.

8. Probeninjektionsverfahren nach Anspruch 1, wobei die Fluidprobe einen löslichen Liganden enthält.

9. Probeninjektionsverfahren nach Anspruch 8, wobei der lösliche Ligand der gleiche Ligand ist, der an mindestens einem der Erfassungsbereiche (18) immobilisiert ist.

10. Probeninjektionsverfahren nach Anspruch 1, wobei die Dispersionsbedingungen umfassen: Injizieren der Fluidprobe bei einer oberen Fließratengrenze, welche größer als 3,0 ist, definiert als eine dimensionslose Zeit.

## Revendications

1. Procédé d'injection d'échantillon pour déterminer les paramètres d'interaction d'affinité d'interactions analyte-ligand dans un biocapteur, le biocapteur comprenant un conduit de canal d'écoulement (14) en communication fluide avec un conduit de cellule d'écoulement (16), ledit conduit de cellule d'écoulement (16) logeant au moins une région de détection (18), ladite région de détection (18) présentant au moins un ligand (19) immobilisé sur son dessus, le procédé comprenant les étapes de :
obtention d'un échantillon de fluide contenant un analyte ayant une concentration de départ ;
utilisation d'une seule injection faisant passer l'échantillon de fluide à travers le conduit de canal d'écoulement (14) jusqu'au conduit de cellule d'écoulement (16) dans soit des conditions d'injection de dispersion Taylor pulsée, soit des conditions d'injection de dispersion Taylor sigmoïdale,
dans lequel dans des conditions d'injection de dispersion Taylor pulsée, l'échantillon de fluide présente un volume qui est inférieur ou égal à 10 % de la capacité totale du conduit de canal d'écoulement (14), et
dans lequel dans des conditions d'injection de dispersion Taylor sigmoïdale, l'échantillon de fluide présente un volume qui est de 50 % à 100 % de la capacité totale du conduit de canal d'écoulement (14) ;
dans lequel lesdites conditions de dispersion résultent en la formation d'un gradient de concentration d'analyte ayant une concentration maximale d'analyte et une concentration minimale d'analyte qui sont différentes d'au moins un ordre de grandeur, et dans lequel la concentration d'analyte dans le gradient de concentration d'analyte change en continu entre la concentration d'analyte maximale et la concentration d'analyte minimale ;
mesure des réponses obtenues par l'analyte interagissant avec le ligand (19) sur la région de détection (18) lorsque le gradient de concentration d'analyte progresse à travers le conduit de cellule d'écoulement (16), dans lequel les réponses mesurées fournissent une courbe de réponse de liaison ;
choix d'un terme de dispersion qui représente la concentration d'analyte présente dans la région de détection (18) à un temps quelconque pendant l'injection, dans lequel dans des conditions de dispersion de Taylor pulsée, le terme de dispersion est représenté par l'Equation 1 $C \left(t\right) = \frac{2 {C}_{in} {V}_{i}}{{π}^{3 / 2} {d}^{2} \sqrt{kt}} exp \left[-\frac{0.25 {L}^{2} {\left(1-t/τ\right)}^{2}}{kt}\right]$ où
| | |
|---|---|
| C(*t*) | = concentration d'analyte au détecteur (mol m⁻³) |
| *Cᵢₙ* | = concentration d'analyse injecté (mol m⁻³) |
| *Vᵢ* | = volume d'injection d'échantillon (m³) |
| *d* | = diamètre de capillaire (conduit de canal d'écoulement) (m) |
| *τ* | = temps de séjour moyen d'analyte (s) ou *L*/*u* |
| *L* | = longueur de capillaire (m) |
| *u* | = vitesse moyenne de fluide (m s⁻¹) |
| *k* | = coefficient de dispersion de Taylor-Aris (m² s⁻¹) qui est représenté par l'équation suivante : |
| $α \frac{{u}^{2} {d}^{2}}{192 D} + D$ | |
où
| | |
|---|---|
| *D* | = coefficient de diffusion d'analyte (m² s⁻¹) et |
| α | = facteur de forme de conduit |
et dans lequel dans des conditions de dispersion de Taylor sigmoïdale, le terme de dispersion est représenté par l'Equation 3 $\begin{matrix}C \left(t\right) = \frac{{C}_{in}}{2} \left[1-erf\frac{1 - \frac{t}{τ}}{2 \sqrt{\frac{k}{uL} \frac{t}{τ}}}\right] & \left(t<τ\right)\end{matrix}$
pour les temps antérieurs au temps nécessaire pour injecter un volume d'échantillon de fluide qui est égal au volume de capacité totale du conduit de canal d'écoulement (14) et l'Equation 4 $\begin{matrix}C \left(t\right) = \frac{{C}_{in}}{2} \left[1+erf\frac{1 - \frac{t}{τ}}{2 \sqrt{\frac{k}{uL} \frac{t}{τ}}}\right] & \left(t>τ\right)\end{matrix}$ où
| | |
|---|---|
| *C(t)* | = concentration d'analyte au détecteur (mol m⁻³) |
| *Cᵢₙ* | = concentration d'analyte injecté (mol m⁻³) |
| *τ* | = temps de séjour moyen d'analyte (s) ou *L*/*u* |
| *L* | = longueur de capillaire (m) |
| *u* | = vitesse moyenne de fluide (m s⁻¹) |
| *k* | = coefficient de dispersion de Taylor-Aris (m² s⁻¹) (*Equation 2*) |
| erf | = fonction d'erreur de Gauss |
pour des temps postérieurs au temps nécessaire pour injecter un volume d'échantillon de fluide qui est égal au volume de capacité totale du conduit de canal d'écoulement (14), dans lequel les Equations 3 et 4 incluent un coefficient de dispersion représenté par l'équation (2) ;
incorporation du terme de dispersion dans un modèle d'interaction de liaison ; et
détermination des paramètres d'interaction de liaison par ajustement du modèle d'interaction de liaison à la courbe de réponse de liaison.

2. Procédé d'injection d'échantillon selon la revendication 1, dans lequel le modèle d'interaction de liaison est un modèle d'interaction cinétique qui permet la détermination de la(des) constante(s) de vitesse d'association et de la(des) constante(s) de vitesse de dissociation, dans lequel le modèle d'interaction de liaison est choisi dans le groupe consistant en un modèle d'interaction de pseudo-premier ordre 1:1 à deux compartiments, un modèle d'interaction de pseudo-premier ordre 1:1 simple, un modèle d'interaction d'analyte hétérogène, un modèle d'interaction de ligand hétérogène, un modèle d'interaction de modification conformationnelle, un modèle de distribution d'analyte, un modèle de distribution de ligand, et un modèle d'interaction de liaison phénoménologique.

3. Procédé d'injection d'échantillon selon la revendication 1, dans lequel le modèle d'interaction de liaison est un modèle d'interaction d'affinité qui permet la détermination de la(des) constante(s) d'affinité et est choisi dans le groupe consistant en un modèle d'interaction de liaison 1:1, un modèle d'interaction d'analyte hétérogène, un modèle d'interaction de ligand hétérogène, un modèle d'interaction de modification conformationnelle, un modèle de distribution d'analyte, un modèle de distribution de ligand, et un modèle d'interaction de liaison phénoménologique.

4. Procédé d'injection d'échantillon selon la revendication 1, dans lequel les conditions de dispersion incluent que l'écoulement d'échantillon de fluide à travers le conduit de canal d'écoulement (14) soit un écoulement laminaire.

5. Procédé d'injection d'échantillon selon la revendication 1, dans lequel les conditions de dispersion incluent le conduit de canal d'écoulement (14) présentant une capacité de volume total d'environ 10 µL à environ 2 000 µL et un diamètre d'environ 0,05 mm à 1,0 mm.

6. Procédé d'injection d'échantillon selon la revendication 1, dans lequel le conduit de cellule d'écoulement (16) comprend de plus une région de détection (18b) exempte de liant immobilisé, dans lequel le procédé comprend de plus les étapes de :
mesure d'une réponse d'indice de réfraction en masse sur la région de détection (18b) exempte de ligand immobilisé produite par le gradient de concentration d'analyte lorsqu'il progresse à travers le conduit de cellule d'écoulement (16), dans lequel les réponses mesurées fournissent une courbe de réponse d'indice de réfraction en masse ;
dans lequel le terme de dispersion comprend un coefficient de dispersion représenté en partie par un coefficient de diffusion de l'analyte ; et
la détermination du coefficient de diffusion de l'analyte par ajustement de la courbe de réponse d'indice de réfraction en masse à l'équation de dispersion.

7. Procédé d'injection d'échantillon selon la revendication 6, dans lequel le terme de dispersion est représenté par l'équation 1 où le terme *C(t)* est remplacé par l'indice de réfraction de l'analyte au moment *(f)* et le terme *Cᵢₙ* est remplacé par l'indice de réfraction en masse maximal de l'analyte, et dans lequel le coefficient de dispersion est représenté par l'Equation 2.

8. Procédé d'injection d'échantillon selon la revendication 1, dans lequel l'échantillon de fluide comprend un ligand soluble.

9. Procédé d'injection d'échantillon selon la revendication 8, dans lequel le ligand soluble est le même ligand qui est immobilisé sur au moins une des régions de détection (18).

10. Procédé d'injection d'échantillon selon la revendication 1, dans lequel les conditions de dispersion comprennent l'injection de l'échantillon de fluide à une limite de débit supérieure qui est supérieure à 3,0 comme définie en termes de temps sans dimension.
